# EUROPEAN PATENT SPECIFICATION

(11) **EP 3 288 541 B1**
(45) Date of publication and mention of the grant of the patent: **02.09.2020**
(21) Application number: 15766292.5
(22) Date of filing: 02.09.2015
(51) Int. Cl.: A61K 9/14, A61K 9/72, A61K 31/167, A61K 31/137, A61K 31/58, A61P 11/00

(54) **MULTIDRUG BRITTLE MATRIX COMPOSITIONS**
SPRÖDMATRIXZUSAMMENSETZUNGEN MIT MEHREREN ARZNEIMITTELN
COMPOSITIONS MATRICIELLES FRAGILES À MÉDICAMENTS MULTIPLES

(30) Priority: 01.05.2015 US 201562156052 P
(43) Date of publication of application: 07.03.2018
(73) Proprietor: Board of Regents, The University of Texas System, Austin, TX 78701 (US)
(72) Inventor: WILLIAMS, Robert, Austin, TX 78746 (US); WATTS, Alan, Austin, TX 78731 (US); PETERS, Jay, San Antonio, TX 78248 (US); RAFFA CARVALHO, Simone, Austin, TX 78712 (US)
(74) Representative: Vossius & Partner Patentanwälte Rechtsanwälte mbB
(86) International application number: PCT/US2015/048093
(87) International publication number: WO 2016/178704

(56) References cited:
- WO-A1-2011/069197
- WO-A1-2013/130767
- WO-A2-2011/057266
- US-A1- 2015 017 248
- Anonymous: "Lecithin", Martindale: The Complete Drug Reference, 12 November 2015 (2015-11-12), pages 1-4, XP055242517, Retrieved from the Internet: URL:https://www.medicinescomplete.com/mc/m artindale/current/596-l.htm?q=lecithin&t=s earch&ss=text&p=1#_hit [retrieved on 2016-01-18]
- SHA LIU ET AL: "Formulation of a novel fixed dose combination of salmeterol xinafoate and mometasone furoate for inhaled drug delivery", EUROPEAN JOURNAL OF PHARMACEUTICS AND BIOPHARMACEUTICS., vol. 96, 26 July 2015 (2015-07-26), pages 132-142, XP055242491, NL ISSN: 0939-6411, DOI: 10.1016/j.ejpb.2015.07.017

## Description

### BACKGROUND OF THE INVENTION

### 1. Field of the Invention

The present invention relates generally to the field of pharmaceutical compositions. More particularly, it concerns pharmaceutical compositions with two active pharmaceutical ingredients prepared as brittle matrix particles.

### 2. Description of Related Art

Asthma is a serious health problem and presents a significant burden to families throughout the world. It places severe limits on daily life and can be fatal. People of all ages are affected by this chronic airway disorder, and the incidence of asthma is increasing in most countries especially among children (Bateman *et al.,* 2008). One of the indicated treatment methods for asthma is corticosteroids (ICS) with or without long acting β2-agonists (LABA) (Silvasti *et al.,* 1996; Chowdhury and Pan, 2010). Additionally, the ICS/LABA combination is an effective therapy for chronic obstructive pulmonary disease (COPD) that, compared to other therapies, has been shown to reduce exacerbations, hospitalizations, emergency room visits and health care costs (Mapel *et al.,* 2010).

The ability of long-acting β2-agonists to prime glucocorticosteroid receptors improves the activity of inhaled corticosteroids. It is important that inhaled combinations are co-deposited within the lungs, since this synergy occurs at the cellular/molecular level (Greening *et al.,* 1994). In addition, salmeterol xinafoate (SX) is sparingly soluble and mometasone furoate (MF) is practically insoluble in water (Jouyban-Gharamaleki *et al.,* 2001; Zitt *et al.,* 2007), and their low solubility limits absorption and bioavailability, which impacts their clinical use. Different crystal engineering strategies have been effective at modifying the physicochemical properties and enhancing oral bioavailability for specific compounds, more recently especially co-crystal (Sowa *et al.,* 2014; Évora *et al.,* 2011) and co-amorphous systems (Lobmann *et al.,* 2013; Shayanfar *et al.,* 2013), which could achieve co-deposition for formulations consisted of more than one API component.

However, presently there is no fixed dose combination product marketed for inhalation containing SX and MF contained within the same particle. Having SX and MF within the same particle allows deposition of the APIs at the same target site within the lungs. Presently available products containing two APIs are typically micronized separately and remain as discrete particles in the formulation and device (Patil *et al.,* 2012). Furthermore, micronized API particles can possess different crystallography and morphology, resulting in differences in aerodynamic performance (Parikh *et al.,* 2012). Researchers have presented a solution to the problem of traditional blends by engineering multiple drugs into a single particle (Parikh *et al.,* 2012; Weers and Tarara, 2014). Thus, new compositions which contain multiple active pharmaceutical ingredients with the same particle are needed.

The dissertation of Simone Raffa Cavalho, University of Texas at Austin, December 2013, relates to inhalation therapies of brittle powders. Triple fixed dose therapy using formoterol fumarate, tiotropium bromide and budesonide as drugs was investigated.

WO 2011/069197 A1 discloses inhalable formulations which may comprise mometasone furoate and salmeterol xinafoate.

### SUMMARY OF THE INVENTION

The present disclosure provides pharmaceutical compositions formulated as a brittle matrix particle for intranasal, via inhalation, via aerosol, or administered to the lungs.

These compositions comprise a long acting β-agonist and a corticosteroid. These compositions may be used to treat a respiratory disease or disorder including but not limited to asthma or chronic obstructive pulmonary disease (COPD).

In some aspects, the present disclosure provides pharmaceutical compositions comprising :
a dual therapy comprising a therapeutically effective amount of the following active pharmaceutical ingredients:
   i. a long acting β-agonist (LABA) which is salmeterol xinafoate, and
   ii. a corticosteroid (CS) which is mometasone furoate;
wherein the pharmaceutical composition is formulated as a brittle matrix particle having a specific surface area of 20 m²/g or greater. In some embodiments, the pharmaceutical compositions further comprise one or more excipients. The excipients that may be used include a sugar, sugar derivative selected from aminosugars and sugar alcohols, or an amino acid. The excipients that may be a sugar or a sugar derivative such as a lactose, mannitol, or trehalose. In other embodiments, the excipients are an amino acid such as glycine.

The dual therapies may comprise a weight ratio of the long acting β-agonist to the corticosteroid from 1:0.1 to 1:100 in the composition. In some embodiments, the dual therapies have a weight ratio of 5:22 of the long acting β-agonist to the corticosteroid.

The pharmaceutical compositions may have a molar ratio of the dual therapy to the excipient of from 1:0 to 1:9 in the composition such as 1:1 of the dual therapy to the excipient.

In some aspects, the pharmaceutical compositions are formulated as a unit dose. The unit dose of the pharmaceutical compositions may comprises a dose of the long acting β-agonist from 1 to 500 µg such as 50 µg of the long acting β-agonist salmeterol xinafoate.

In some embodiments, the unit dose of the pharmaceutical compositions comprises a dose of the corticosteroid from 1 to 1000 µg such as 220 µg of the corticosteroid mometasone furoate.

In some aspects, the pharmaceutical compositions re formulated for administration: intranasally, via aerosol, to the lungs, or via inhalation. The pharmaceutical compositions may be formulated for use in an inhaler including a metered dose inhaler, a dry powder inhaler, a single dose inhaler, a multi-unit dose inhaler, a nebulizer, or a pressurized metered dose inhaler.

In some embodiments, the pharmaceutical compositions have a specific surface area from 20 m²/g to 250 m²/g. In some embodiments, the pharmaceutical compositions have a total emitted dose (TED) of greater than 85% such as from 90% to 100%.

In some embodiments, the pharmaceutical composition is free of any impurities. The pharmaceutical composition may be substantially free of polyvinylpyrrolidone, polyvinylalcohol, polyacrylate, or polystyrene. The pharmaceutical composition may be essentially free of polyvinylpyrrolidone, polyvinylalcohol, polyacrylate, or polystyrene. In some embodiments, the pharmaceutical composition is essentially free of any polymeric excipients. The pharmaceutical composition may be substantially free of poloxamers, polyethylene glycol, or polypropylene glycol. The pharmaceutical composition may be essentially free of poloxamers, polyethylene glycol, or polypropylene glycol. In some embodiments, the pharmaceutical composition is essentially free of any surfactants. In some embodiments, the pharmaceutical composition is free of other compounds beyond the excipient and the active pharmaceutical composition.

The pharmaceutical compositions of the present invention can be used in methods of treating or preventing a respiratory disease or disorder in a patient in need thereof comprising administering to the patient a therapeutically effective amount of a pharmaceutical composition disclosed herein. The respiratory diseases or disorders that may be treated include a disorder involving inflammation of the lungs or sinuses. Additionally, the respiratory disease or disorder may be asthma or chronic obstructive pulmonary disease. In some embodiments, the pharmaceutical composition is administered via inhalation. The therapeutically effective amount may be administered to the patient in one inhalation, or in 2 or more inhalations. In some embodiments, the therapeutically effective amount is administered in 2, 3, or 4 inhalations. In some embodiments, the method comprises administering the therapeutically effective amount to the patient once a day. In other embodiments, the method comprises administering the therapeutically effective amount to the patient two or more times a day.

In still yet another aspect, the present disclosure provides methods of preparing a brittle matrix pharmaceutical composition comprising:
(A) admixing two active pharmaceutical agents into a solvent wherein the solvent comprises an organic solvent and water to form a pharmaceutical composition wherein the pharmaceutical composition comprises an amount of the active pharmaceutical agents in the solvent from 0.01% to 10% (w/v);
(B) applying the pharmaceutical composition to a rotating surface wherein the surface is at a temperature from -70 °C to -120 °C; and
(C) freezing the pharmaceutical composition to form a brittle matrix pharmaceutical composition, which has a specific surface area of 20 m²/g or greater.

The active pharmaceutical ingredients are salmeterol xinafoate as a long acting β-agonist, and momatesone furoate as a corticosteroid. The methods may also further comprise adding one or more excipients to the pharmaceutical composition. In some embodiments, the method further comprises lyophilizing the brittle matrix pharmaceutical composition. In some embodiments, the amount is from 0.01% (w/v) to 6% (w/v). In some embodiments, the amount is from 0.1% (w/v) to 5% (w/v).

As used herein the specification, "a" or "an" may mean one or more. As used herein in the claim(s), when used in conjunction with the word "comprising", the words "a" or "an" may mean one or more than one.

As used herein "another" may mean at least a second or more.

Throughout this application, the term "about" is used to indicate that a value includes the inherent variation of error for the device, the method being employed to determine the value, or the variation that exists among the study subjects.

The term "effective," as that term is used in the specification and/or claims, means adequate to accomplish a desired, expected, or intended result. "Effective amount," "Therapeutically effective amount" or "pharmaceutically effective amount" when used in the context of treating a patient or subject with a compound means that amount of the compound which, when administered to a subject or patient for treating a disease, is sufficient to effect such treatment for the disease.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

The term "free of" is used to imply a particle which contains at least 95% of the listed components and less than 5% of the components to which composition or particle is free of. The term "substantially free of" is used to 98% of the listed components and less than 2% of the components to which composition or particle is substantially free of. In some embodiments, the term "essentially free of" is used to describe a particle or composition which contains at least 99% of the listed components and contains less than 1% of any components of the components to which it is essentially free of.

As used herein the term dual therapy comprises at least the two active pharmaceutical ingredients listed therein. It is also contemplated that these terms are not meant to imply that these therapies comprise only two active pharmaceutical ingredients but rather that they comprise at least two active pharmaceutical ingredients.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
FIG. 1 shows geometric particle size distribution of BMP before and after dosing from a HandiHaler®.
FIG 2 shows aerodynamic particle size distribution of a triple combination aerosol using micronized (left) and BMP (right) formulations with the values of formoterol, tiotropium, and budesonide shown from left to right for each site. Aerosols were generated using a HandiHaler® at 51 l/min.
FIG. 3 shows modulated DSC profiles (from bottom to top) BMP SXMFTre, BMP SXMFGly, BMP SXMFMan, BMP SXMFLac and BMP SXMF. T_{g} was shown in small window.
FIGS. 4A & 4B show powder X-ray diffraction patterns for the compositions. FIG. 4A shows the X-ray powder diffraction patterns for (from bottom to top): micronized SXMF, BMP SXMF, micronized SXMFLac, BMP SXMFLac, micronized SXMFMan, BMP SXMFMan, micronized SXMFGly, BMP SXMFGly, micronized SXMFTre, TFF SXMFTre. FIG. 4B shows the X-ray powder diffraction patterns of BMP combinations stored at 25°C/30%RH for 6 months (from bottom to top): BMP SXMF, BMP SXMFLac, BMP SXMFMan, BMP SXMFGly and BMP SXMFTre.
FIGS. 5A & 5B show the SEM images of (FIG. 5A) Micronized SXMF (FIG. 5B) BMP SXMF.
FIGS. 6A & 6B show the Fourier transform infrared spectroscopy of (FIG. 6A) micronized single ingredient (FIG. 6B) TFF processed co-drug deposition amorphous forms.
FIG. 7 shows the isotherms of sorption (-) and desorption (---) of (□) BMP SXMF, (◇) BMP SXMFLac, (Δ) BMP SXMFMan, (×) BMP SXMFGly and (○) BMP SXMFTre produced after one cycle between 0% and 90%.
FIGS. 8A-8E show the aerodynamic diameter distribution of SX and MF for (FIG. 8A) micronized SXMF and BMP SXMF, (FIG. 8B) micronized SXMFLac and BMP SXMFLac, (FIG. 8C) micronized SXMFMan and BMP SXMFMan, (FIG. 8D) micronized SXMFGly and BMP SXMFGly, and (FIG. 8E) micronized SXMFTre and BMP SXMFTre after produced by Miat at 90 L/min. Values were expressed as mean ± SD (n=3).
FIG. 9 shows the aerodynamic diameter distribution of SX and MF for micronized SXMF and TFF SXMF after produced by Insufflator at 51 L/min.

### DESCRIPTION OF ILLUSTRATIVE EMBODIMENTS

In some aspects, the present disclosure provides a pharmaceutical composition formulated as a brittle matrix particle with two active pharmaceutical ingredient (API) for administration via inhalation, via aerosol, or other methods of delivering the compound to the lungs. The pharmaceutical compositions described herein may comprise a high surface area and a high total emitted dose. In some embodiments, these pharmaceutical compositions have increased effectiveness in delivering two active pharmaceutical ingredients to the lungs of a patient.

### I. Brittle Matrix Particles

In some aspects, the present disclosure provides the use of brittle matrix particles (BMP). The brittle matrix particles that may be used herein are characterized by their low density configuration with a high surface area and high porosity. These brittle matrix particles may be prepared using convention methods such as spray freeze drying or thin film freezing as described herein and in U.S. Patent Application No. 2010/0221343 and Watts, *et al.,* 2013. In some embodiments, thin film freezing is used to prepare the brittle matrix particles described herein. After freezing, these particles may be further subjected to drying to obtain a dry powder suitable for aerosol administration. The brittle matrix particles may be dried through lyophilization and other methods known to those of skill in the art. Without wishing to be bound by any theory, the brittle matrix particles and the fast freezing drying methods allow the mixing of the particles while maintaining the homogeneity of the mixture wile preventing segregation of the different components. The improved homogeneity may also be exhibited during the aerosolization process.

In some aspects, the brittle matrix particles are prepared using thin film freezing (TFF) methods. Such preparation may be used in a manner to allow for the co-deposition of two active pharmaceutical ingredients (APIs) and one or more excipients to form a pharmaceutical composition. In some embodiments, the methods comprise dissolving the pharmaceutical composition in a solvent. Some solvents which may be used in the methods described herein include water, an organic solvent, or a mixture thereof. The organic solvents that may be used herein include polar organic solvents such an alcohol, a heterocyclic compound, an alkylnitrile, or a mixture thereof. Some non-limiting examples of polar organic solvents include methanol, ethanol, isopropanol, tert-butanol (tertiary butanol), dimethylsulfoxide, dimethylformamide, 1,4-dioxane, or acetonitrile. In some aspects, mixtures of these solvents are contemplated. Such mixtures may comprise one or more organic solvents with water. One non-limiting example of these mixtures includes the solvent mixture of tert-butanol, 1,4-dioxane, acetonitrile, and water. The solvent mixture may comprise a mixture of tertiary butanol, 1,4-dioxane, acetonitrile, and purified water in a ratio of 2:1:3:3 (v/v).

In some aspects, the present disclosure comprises a combination of two active pharmaceutical ingredients (APIs). These combinations may further comprise one or more excipients. Some non-limiting examples of some excipients which may be used herein include a sugar or sugar derivative, such as mannitol, trehalose, or lactose, or an amino acid, such as glycine. These compositions may be dissolved in a solvent as described herein.

The active pharmaceutical ingredients (APIs) are a long acting β-agonist (LABA) which is salmeterol xinafoate, and an inhaled corticosteroid (ICS) which is mometasone furoate.

The agents may be present in a ratio for the LABA to the ICS of from 1:0.1 to 1:100. In some embodiments, the ratio of the LABA to the ICS is 1:4.4. This ratio produces an amount of each component in an effective clinical dose.

In some aspects, the pharmaceutical composition comprises an excipient. In other aspects, the active pharmaceutical ingredient is formulated in the pharmaceutical composition without an excipient. When the composition comprises an excipient, the excipient may be present from about no excipient to a molar ratio of 1:9 active pharmaceutical ingredients to the excipient. In some embodiments, the molar ratio of active pharmaceutical ingredients to excipients is from about a composition comprising no excipient to a molar ratio comprising 1:1 ratio of active pharmaceutical ingredients to excipients. The molar ratio of active pharmaceutical ingredients to excipients may be 1:1.

The composition may be dissolved in a solvent as described above. When the composition is dissolved in a solvent, the total amount of the pharmaceutical composition in the solvent may be from 0.1% to 10% (w/v). The total amount of the pharmaceutical composition may be from 0.1% to 6% (w/v). In some aspects, the total amount of the pharmaceutical composition is less than 6%, 5%, 4%, 3.5%, 3%, 2.5%, 2.0%, 1.75%, 1.5%, 1 1.25%, 1.0%, 0.9%, 0.8%, 0.7%, 0.6%, 0.5%, 0.4%, 0.3%, 0.2%, or 0.1%, or any range derivable therein. The total amount of the pharmaceutical composition in the solvent is preferable less than 6%, more preferably less than 5%. Using small amount of the pharmaceutical composition in the solvent is believed to give the advantageous properties such as leading to the formation of a brittle matrix particle and thus using less than 6% (w/v) and preferably less than 5% (w/v) is recommended. While lower amounts of the compounds are beneficial, the concentrations below 0.01% (w/v) or more preferably 0.1% (w/v) may lead to solutions too dilute to obtain a useful pharmaceutical composition. In some embodiments, the total amount of the pharmaceutical composition is about 0.5% (w/v).

In some aspects, the compositions are prepared using a thin film apparatus. The apparatus may be used to apply the solution to a surface such as a stainless steel and then frozen. This surface may also be rotating such that without wishing to be bound by any theory, it is believed that the rotating prompts the even application of the solution to the surface. The solution may be frozen at a cryogenic temperature such as a temperature below - 50 °C. Cryogenic temperatures include a temperature from - 50 °C to - 270 °C, from - 70 °C to - 120 °C, or from - 75 °C to - 100 °C. In some embodiments, the cryogenic temperature is 90 °C ± 3 °C. In some aspects, the samples are stored frozen. In other aspects, the samples are lyophilized to obtain a dry powder. Lyophilization is known to those of skill in the art and is taught in U.S. Patent Nos. 5,756,468, 6,440,101, 8,579,855, and PCT Patent Application Publication No. WO 2009/125986. In some aspects, it may be advantageous to store the composition at room temperature. The lyophilized samples may be prepared such that the temperature is gradually increased from the lyophilization temperature of less than - 40°C to a temperature around room temperature such as 25 °C. Also, the increase in temperature may be carried out under a vacuum or in a reduced pressure environment and/or an environment which has a reduced moisture content such as a desiccator.

The present disclosure provides brittle matrix particles which have a high surface area compared to other techniques such as jet milling or physical mixtures.

The brittle matrix particles may have a specific surface area from 20 m²/g to 250 m²/g. In some embodiments, the specific surface area is from 20, 25, 30, 35, 40, 45, 50, 60, 70, 80, 90, 100, 125, 150, 175, 200, 225, 250, 300, 350, 400, 450, 500, 600, 700, 800, 900, to 1000 m²/g, or any range derivable therein. Methods of calculation the surface area of the composition are described herein in the Examples sections.

The brittle matrix particles comprising a pharmaceutical composition described herein may have a total emitted dose (or emitted dose) of greater than 80% of the active pharmaceutical ingredient. The total emitted dose may also be from 80% to 100%, from 85% to 100%, or from 90% to 100%. The formulations of the pharmaceutical composition described herein may have an total emitted dose of greater than 80%, 81%, 82%, 83%, 84%, 85%, 86%, 87%, 88%, 89%, 90%, 91%, 92%, 93%, 94%, 95%, 96%, 97%, 98% or 99%, or any range derivable therein. Methods of calculating the total emitted dose are described herein in the Examples section.

The pharmaceutical compositions described herein may comprise one or more excipients. Excipients are components which are not therapeutically active but may be used in the formation of a pharmaceutical composition. The excipients used herein include amino acids, sugars, sugar derivatives, or other excipients know those of skill in the art. In particular, the present disclosure includes the use of a sugar such as trehalose, lactose, glucose, fructose, or mannose, or a sugar derivative selected from an aminosugar such as glucosamine and a sugar alcohol such as mannitol. Other excipients which may be used include amino acids such as alanine or glycine.

In some aspects, the brittle matrix particles component contains two or more drug molecules with one or more excipients to form a pharmaceutical composition. The pharmaceutical composition can thus be formulated in the brittle matrix particles in an amorphous form or in a particular crystalline form. In some embodiments, the pharmaceutical composition is formulated in the amorphous form. Additionally, the brittle matrix particles that may be used are a low density particle.

The present disclosure provides methods which makes use of the brittle matrix particles in the aerosol administration of a pharmaceutical composition. Without wishing to be bound by any theory, it is believed that the brittle matrix particles are readily fractured during the aerosolization thus enhancing the delivery of the pharmaceutical composition. The fracturing of the particles may be used to enhance the composition's ability to aerosolize and dispersion during administration.

In certain embodiments, the pharmaceutical compositions may be delivered by inhalation and/or other aerosol delivery vehicles. Methods for delivering compositions directly to the lungs via nasal aerosol sprays has been described e.g., in U.S. Pat. Nos. 5,756,353 and 5,804,212 . Likewise, the delivery of drugs using intranasal microparticle resins (Takenaga *et al.,* 1998) and lysophosphatidyl-glycerol compounds (U.S. Pat. No. 5,725, 871) are also well-known in the pharmaceutical arts and may be used in some embodiments. Transmucosal drug delivery in the form of a polytetrafluoroetheylene support matrix is described in U.S. Pat. No. 5,780,045 .

In some embodiments, a brittle matrix particle composition comprising active agents or drugs as described herein may be delivered intranasally, to the lungs, or via inhalation or aerosol delivery. In some embodiments the brittle matrix particle composition comprising active agents or drugs may be delivered via an inhaler (also called a puffer) or a metered dose inhaler. Inhaler apparatuses are well known in the art and may be used to for the delivery of drugs for the treatment of asthma or COPD.

The term aerosol refers to a colloidal system of finely divided solid of liquid particles dispersed in a liquefied or pressurized gas propellant. The typical aerosol of the present invention for inhalation will consist of a suspension of active ingredients in liquid propellant or a mixture of liquid propellant and a suitable solvent. Suitable propellants include hydrocarbons and hydrocarbon ethers. Suitable containers will vary according to the pressure requirements of the propellant. Administration of the aerosol will vary according to subject's age, weight and the severity and response of the symptoms.

### II. Active Pharmaceutical Ingredients

In some aspects, the present disclosure provides pharmaceutical compositions formulated as a brittle matrix particle comprising two active pharmaceutical ingredients. These active pharmaceutical ingredients are a long acting β-agonist which is salmeterol xinafoate, β-agonist, and a corticosteroid which is mometasone furoate.

These active pharmaceutical ingredients may be present in the pharmaceutical compositions described herein according to the ratios described in Table 1.

**Table 1: Dosing and Ratio of Composition of Active Pharmaceutical Ingredient in the Compositions Described Herein**

| **Active Pharmaceutical Ingredient (API)** | **Ratio** | **Dosing** |
|---|---|---|
| **Long Acting β-agonist** | 1 | about 1 to about 500 µg |
| **Corticosteroid** | about 0.1 to about 100 | about 1 to about 1000µg |

In some aspects, the compositions used herein may comprise a ratio of the long acting β-agonist to the corticosteroid from 1:0.1 to 1:100.

Non-limiting example of the ratio used in dual therapy includes 1:4.4.

In some aspects, the pharmaceutical compositions comprise a weight percentage of the active pharmaceutical ingredients in the composition from 10 wt% to 100 wt%.

These pharmaceutical compositions may also be formulated as a unit dose with a dose of the long acting β-agonist from 1 µg to 500 µg and a dose of the corticosteroid from 1 µg to 1,000 µg.

The long acting β-agonist may be formulated in a dose from about 1 µg, 5 µg, 10 µg, 20 µg, 40 µg, 60 µg, 80 µg, 100 µg, 120 µg, 140 µg, 160 µg, 180 µg, 200 µg, 225 µg, 250 µg, 275 µg, 300 µg, 325 µg, 350 µg, 400 µg, 450 µg, or 500 µg or any range derivable therein.

The corticosteroid may be formulated in a dose from 1 µg, 5 µg, 10 µg, 25 µg, 50 µg, 75 µg, 100 µg, 150 µg, 200 µg, 250 µg, 300 µg, 350 µg, 400 µg, 450 µg, 500 µg, 550 µg, 600 µg, 650 µg, 700 µg, 750 µg, 800 µg, 850 µg, 900 µg, 950 µg, or 1,000 µg or any range derivable therein. The specific dose of each active pharmaceutical ingredient will vary depending on the specific compound present in the pharmaceutical composition.

### III. Respiratory Indications

In some aspects, the compositions of the present disclosure are used in the treatment of respiratory diseases and conditions. Some non-limiting examples of respiratory disease and conditions include asthma, acute respiratory distress syndrome (ARDS), chronic pulmonary inflammatory disease (COPD), reactive airways dysfunction syndrome (RADS), airway hyperreactivity, eosinophilic or interstitial lung disease such as sarcoidosis or eosinophilic granulomatosis, chemical-induced lung injury, plastic bronchitis, bronchitis, chronic bronchitis, chronic obstructive pulmonary (airway) disease, silicosis, inhalational smoke induced acute lung injury (ISALI), or immune diseases and conditions such as allergic rhinitis and chronic sinusitis. The pharmaceutical compositions described herein may be used for the treatment of respiratory conditions such as asthma and chronic obstructive pulmonary disease and other obstructive airways diseases.

Chronic obstructive pulmonary disease (COPD) is a disease which is characterized by clinically poor airflow such as reduced which does not improve over several months. Cigarette smoking including long term cigarette smoking is believed to be the leading cause of COPD. Airflow obstruction in COPD is usually progressive in patients who continue to smoke eventually leading to disability and shortened survival time. Smoking cessation has been shown to slow the rate of decline to that of a non-smoker but the damage caused by smoking is irreversible. Other etiological factors (e.g., airway hyper responsiveness or hypersensitivity), air pollution (e.g., sulfur dioxide and possibly second hand smoke), occupational chemicals (e.g., cadmium) and generally allergy) have been identified in the literature but are believed to account for only a minority of COPD cases. Other risk factors include: heredity, second-hand smoke, exposure to air pollution at work and in the environment, and a history of childhood respiratory infections.

Some non-limiting examples of COPD include chronic coughing, frequent chest tightness, shortness of breath, an increased effort to breathe, increased mucus production, and frequent clearing of the throat. In some instances of COPD, airway obstruction is incompletely reversible, but other COPD patients do show some improvement in airway obstruction with treatment. Airway obstruction due to chronic and excessive secretion of abnormal airway mucus, inflammation, bronchospasm, and infection are believed to cause chronic bronchitis leading to chronic cough, mucus production or both. In emphysema instead, the elastin in the terminal bronchioles is destroyed leading to the collapse of the airway walls and inability to exhale. Emphysema is characterized by the destruction of the alveoli and the abnormal permanent enlargement of the air spaces distal to the terminal bronchioles, accompanied by destruction of their walls without apparent fibrosis.

Asthma is a chronic respiratory disease associated with increased inflammation particular in the airways. Some non-limiting examples of asthma symptoms include wheezing, coughing, chest tightness, shortness of breath, and increased difficulty in breathing. Asthma is generally not considered a type of COPD as asthma is generally reversible. The cough may result in the production of sputum including sputum with high levels of eosinophil. The frequency and severity of asthma symptoms may be greatly increased in the presence of triggers. Some non-limiting examples of asthma triggers include exercise, dust, pollution, pet dander, and other irritants and allergens. Asthma is generally diagnosed by observation of the pattern of symptoms, response to treatment, and spirometry. In some embodiments, there are several specific subtypes of asthma such as occupational, cough-variant, exercise-induced, aspirin-induced, or alcohol induced asthma. Another non-limiting example of a sub-type of asthma is brittle asthma which is characterized by recurrent and severe attacks. Furthermore, asthma may be associated or have its severity increased by other respiratory conditions such as bronchitis or allergies.

Clinical classification of asthma is assigned based upon the frequency of symptoms, the forced expiratory volume in one second (FEV₁), and peak expiratory flow rate or the asthma may be classified by its origin (atopic or extrinsic and topic or intrinsic). Atopic or extrinsic asthma typically is precipitated by allergens or other external factors, while topic or intrinsic asthma is not affected by allergens or other external factors. Additionally, asthma classifications based upon frequency or severity of symptoms. The clinical classifications include intermittent, mild persistent, moderate persistent, and severe persistent. For the intermittent classification, the patient generally experiences less than 2 incidents of symptoms per week, less than 2 nighttime incidents of symptoms per month, a FEV₁ of greater than or equal to 80%, with less than 20% variability, or require the use of a short acting β agonist two or less times per day. For the mild persistent classification, the patient generally experiences less than 2 incidents of symptoms per week, 3 to 4 nighttime incidents of symptoms per month, a FEV₁ of greater than or equal to 80%, with 20-30% variability, or require the use of a short acting β agonist more than two times per day. For the moderate persistent classification, the patient experiences symptoms daily, more than 1 nighttime experiences of symptoms per week, a FEV₁ from about 60% to about 80%, with greater than 30% variability, or require the use of a short acting β agonist daily. For the severe persistent classification, the patient experiences symptoms continuously, frequent nighttime experiences of symptoms (usually greater than 7 times per week), a FEV₁ of less than 60%, with greater than 30% variability, or require the use of a short acting β agonist more than twice daily.

Furthermore, asthma attacks or asthma exacerbations are classified as mild, moderate, or severe based upon the peak expiratory flow rate. For a mild attack, the peak expiratory flow rate is greater than 50% of the predicted best or greater than 200 L/min. For a moderate attack, the peak expiratory flow rate is between 25% and 50% of the predicted best or between 80 and 200 L/min. For a severe attack, the peak expiratory flow rate is less than 25% of predicted best or less than 80 L/min. Additionally, severe attacks may also have one or more additional symptoms or conditions. Severe attacks may be further sub-divided into three categories: acute, life-threatening, or near-fatal. For acute severe attacks, a patient may experience a respiratory rate greater than 25 breaths per minute, a heart rate of greater than 110 beats per minute, or be unable to speak a complete sentence in one breath. For life-threating severe attacks, a patient may experience altered levels of consciousness, exhaustion, arrhythmia, low blood pressure, cyanosis, a silent chest, poor respiratory effect, oxygen saturation of less than 92%, a PaO₂ of less than 8 kPa, or a normal to elevated PaCO₂. For near fatal severe attacks, a patient may experience either a high or elevated PaCO₂ or requires manual ventilation.

Asthma, chronic obstructive pulmonary disease and other respiratory diseases and disorders may be treated with β adrenergic receptor agonists as those compounds are known to provide a bronchodilator effect to the patients, resulting in relief from the symptoms of breathlessness. While β agonists may be used to provide symptomatic relief of bronchoconstriction in patients, another component of asthma or chronic obstructive pulmonary disease is inflammation, which often necessitates separate treatment. One non-limiting method of treating the inflammation caused by these respiratory diseases is through the use of steroids or corticosteroids. In some aspects, the administration of combinations of steroids, β adrenergic receptor agonists, and muscarinic antagonists in a single composition has shown an enhanced, synergistic, effect in terms of treatment of bronchoconstriction, inflammation and mucous secretions of airways in respiratory disease such as asthma and chronic obstructive pulmonary disease.

### IV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1 - Methods

Two different combination therapies were produced by TFF, a cryogenic particle engineering technology (Yang *et al.,* 2012), and characterized for their aerodynamic properties. The first, a dual combination therapy of salmeterol xinafoate (SX) and mometsone furoate (MF) was produced in a 5:22 SX to MF mass ratio. BMP formulations were produced as neat powders as well as with stabilizing excipients, lactose and mannitol, where drug loading totaled 50%. The second drug combination therapy produced by TFF (not according to the claims) was a triple combination of formoterol fumarate (FF), tiotropium bromide (TB), and budesonide (B) in a 1:2:35.5 FF:TB:B mass ratio. Mannitol was included as a stabilizing excipient so that the total drug loading was 50%. For comparison, in both dual and triple combinations, micronized drug was produced (not according to the claims) and blended with micronized excipient. Dry powder aerosols were generated from a HPMC capsule loaded into a HandiHaler® or Monodose® dry powder inhaler. Aerosols were characterized by Next Generation Pharmaceutical Impaction (NGI) operated at flows sufficient for a 4 kPa pressure drop across the device. This was equivalent to 51 L/min for the HandiHaler® and 90 L/min for the Monodose®.

### Example 2 - Results

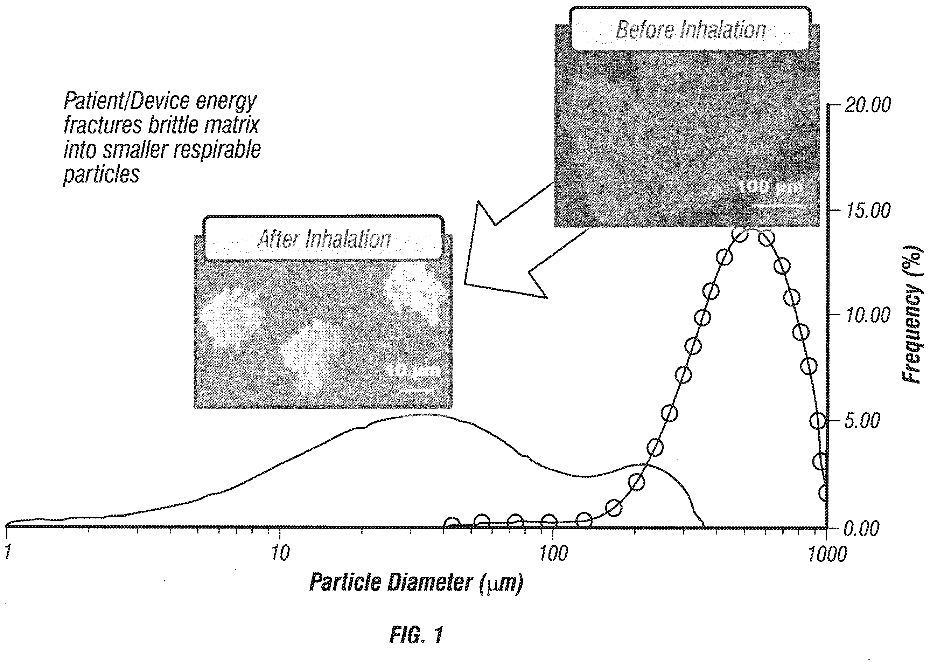

Particle engineering technologies, such as spray drying and TFF, allow for the formulation of multiple actives into a single inhalable particle. BMP created by TFF presents a paradigm shift in dry powder inhalation. As illustrated in FIG. 1, rather than using the inspiratory energy to deagglomerate discrete particles, this platform uses the energy generated by a patient/DPI for the brittle fracture of BMP into respirable low-density particles.

Since TFF is a bottom-up production method that begins with a solution, API and excipients are initially homogeneous at the molecular-level before application to a cryogenic surface for rapid freezing. Using this unique approach to DPI formulation, combinations therapies are homogenously dispersed within aerosol particles resulting in delivered dose uniformity and co-deposition of each API within a combination product. The data presented in Table 2 shows that the aerosol properties of SX and MF in BMP combination formulations are very similar for both APIs. Conversely, micronized powders of SX and MF (not in accordance with the claims) delivered in a combination powder blend demonstrated different fine particle fractions (FPF) and mass median aerodynamic diameters (MMAD). Additionally, BMP SX/MF formulation without excipients (neat) was able to deliver nearly 50% of the both loaded drugs as fine particles.

**Table 2: Aerodynamic properties of SX/MF combination BMP formulations and Micronized formulations delivered by a Monodose® inhaler.**

| Formulation | FPF (% of deliv.) | FPF (% of loaded) | MMAD (µm) | GSD (µm) | TED (%) |
|---|---|---|---|---|---|
| Neat BMP SX/MF | | | | | |
| SX | 55.52±4.78 | 52.90±4.18 | 3.58±0.32 | 3.55±1.12 | 95.31±1.13 |
| MF | 52.08±4.40 | 48.91±4.16 | 3.69±0. 26 | 3.33±0.63 | 93.91±1.27 |

| BMP Mannitol SX/MF | | | | | |
|---|---|---|---|---|---|
| SX | 44.60±4.09 | 42.74±3.22 | 4.45±0.46 | 3.67±1.29 | 95.92±2.07 |
| MF | 43.91±3.37 | 41.06±3.47 | 4.43±0.36 | 3.86±1.53 | 93.49±1.70 |

| Neat Micronized SX/MF | | | | | |
|---|---|---|---|---|---|
| SX | 59.51±7.07 | 40.98±6.77 | 3.30±0.62 | 1.91±0.06 | 68.59±3.57 |
| MF | 34.29±5.78 | 24.50±4.91 | 4.45±0.96 | 2.01±0.02 | 71.18±3.12 |

| Micronized Mannitol SX/MF | | | | | |
|---|---|---|---|---|---|
| SX | 57.52±7.91 | 44.74±6.82 | 3.41± 0. 65 | 1.94±0.07 | 77.67±1.66 |
| MF | 30.07±6.20 | 23.84±5.05 | 4.84±0.92 | 1.94±0.02 | 79.20±0.57 |

| Micronized Lactose SX/MF | | | | | |
|---|---|---|---|---|---|
| SX | 56.15±9.58 | 41.39±8.60 | 3.65±0.58 | 1.00±0.01 | 73.88±1.14 |
| MF | 27.27±6.01 | 21.09±4.37 | 4.84±0.81 | 1.90±0.04 | 77.49±3.46 |

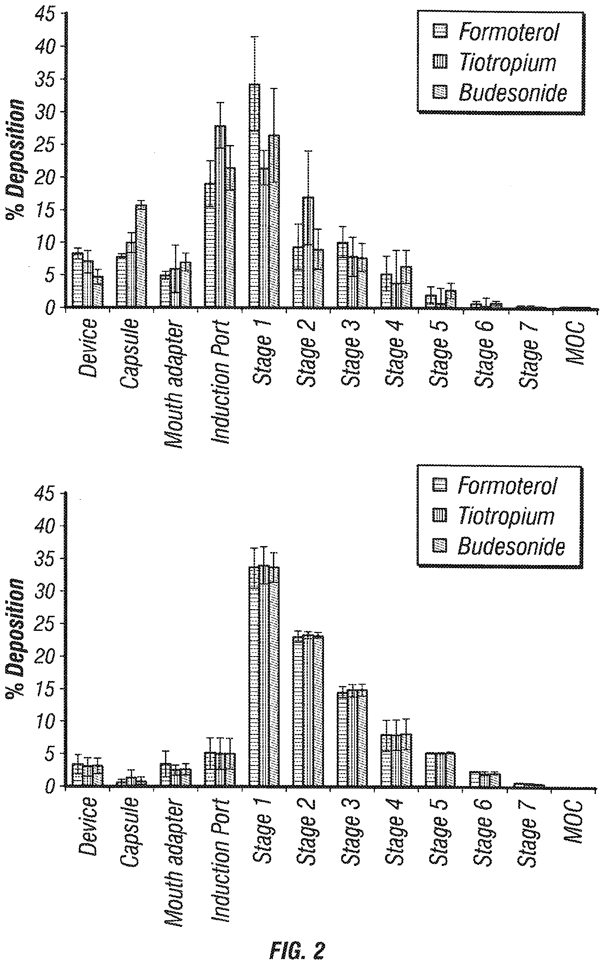

Assessment of the aerodynamic performance of a BMP triple combination of FF, TB and B (not in accordance with the claims) produced similar results to the BMP SX/MF formulation. BMP formulation ensured that individual APIs were delivered and deposited in a homogenous manner. As evident in FIG. 2, the regional deposition of the BMP formulation was nearly identical for all three APIs. This BMP triple combination could improve therapy in patients where β2-agonists receptors are downregulated and long-acting muscarinic antagonist (LAMA) sensitivity is increased (Williamson *et al.,* 2010). While FPF as a percentage of loaded dose was above 50% for all three BMP formulated APIs, further optimization of formulation/device is needed to reduce upper airway (stage 1 and 2) deposition in these formulations.

### Example 3 - Materials and Methods

### A. Material

Salmeterol xinafoate and mometasone furoate were purchased from Mascot I.E.CO., LTD (Changzhou, China). Alpha-lactose monohydrate and mannitol were purchased from Fisher Scientific (NJ, USA); D-Trehalose anhydrous was purchased from Acros Organics (NJ, USA) and glycine was purchased from J.T. Baker (PA, USA). High performance liquid chromatography (HPLC) grade acetonitrile and methanol were purchased from Fisher Scientific (NJ, USA). Water was purified by reverse osmosis (MilliQ, Millipore, France).

### B. Formulation preparation

Thin Film Freezing technology was used to produce BMP formulations for co-deposition. In brief, salmeterol xinafoate (SX), mometasone furoate (MF) and pharmaceutical excipients were dissolved in a co-solvent mixture of tertiary butanol, 1,4-dioxane, acetonitrile and purified water (2:1:3:3, v/v) (Jouyban-Gharamaleki *et al.,* 2001. Combinations of a.) salmeterol xinafoate, mometasone furoate and lactose (SXMFLac), b.) salmeterol xinafoate, mometasone furoate and mannitol (SXMFMan), c.) salmeterol xinafoate, mometasone furoate and glycine (SXMFGly), d.) salmeterol xinafoate, mometasone furoate and trehalose (SXMFTre) and e.) salmeterol xinafoate, mometasone furoate (SXMF) without an excipient, were dissolved in the co-solvent solution. The ratio of salmeterol xinafoate to mometasone furoate was 50 to 220 by mass that corresponded to the clinical dose; while the ratio of APIs to excipient was controlled at a 1:1 molar ratio. Depending on the solubility, the total solid concentration of the co-solvent was 0.5% (w/v). The co-solvent solution was rapidly frozen on a cryogenically cooled (-90 ± 3 °C) rotating stainless steel surface of the thin film apparatus. After removal by a scraper, the frozen films were then collected in a container filled with liquid nitrogen to maintain the frozen state. The frozen samples were transferred to a VirTis Advantage Lyophilizer (VirTis Company Inc., Gardiner, NY) to obtain dry powders. The formulations were lyophilized over 24 h at -40 °C at a pressure less than 27 Pa (200 mTorr), and then the shelf temperature was gradually increased to 25 °C over another 24 h. The final product was stored in a vacuum desiccator at room temperature.

For comparison purposes, the micronized crystalline physical blends of SX, MF and excipients, in the same ratio as used in the BMP formulations, were prepared by jet milling (not in accordance with the invention). Single bulk material was fed into an air-jet mill (Aljet mill, Fluid Energy, Plumsteadville, PA, USA) with a feed pressure of 552 kPa (80 psi) and a grinding pressure of 448 kPa (65 psi). Each bulk material was processed until the particle size reached the respirable range (1-5µm) (Watts *et al.,* 2013), and this varied between 5-7 times. Samples were collected and analyzed from the collecting chamber. The particle size before and after milling was measured using a Sympatec Helos laser diffraction instrument (Sympatec GmbH, Germany) equipped with a R3 lens. Equivalent amounts of micronized SX, MF and excipients were accurately weighed and mixed separately using the geometric dilution technique, and then placed into a stainless steel mixing vessel, blended by Turbula Blender T2F (Bachofen, Switzerland) for 30 min at 48 revolutions per minute (rpm).

### C. Differential scanning calorimetry

Thermal analysis of BMP SXMF, BMP SXMFLac, BMP SXMFMan, BMP SXMFGly, BMP SXMFTre and each of their micronized components were conducted using modulated temperature DSC (Model 2920, TA Instruments, New Castle, DE) equipped with refrigerated cooling system, coupled with TA Universal Analysis 2000 Software (New Castle, DE). Calibration of the DSC instrument was carried out using indium as a standard. Sample powders (5-10 mg) were loaded in an aluminum pan and press-sealed with an aluminum lid (PerkinElmer, Waltham, MA). A crimped empty pan was used as reference. The mass of each empty sample pan was matched with the mass of empty reference pan to ±0.1 mg. Melting endotherm was analyzed at a heating ramp rate of 10 °C/min and modulation temperature amplitude of 1 °C in range of 30-350 °C under nitrogen gas flow rate of 40 mL/min through DSC cell. The glass transition temperature (T_{g}) of BMP formulations was measured by modulated DSC but at a heating ramp rate of 5 °C/min.

### D. Powder X-Ray Diffraction

The morphology of the powder samples was evaluated by wide angle XRD (Rigaku R-Axis Spider, Japan) with an image plate detector using a graphite monochromator with CuKα radiation (λ= 1.5418Å). An acceleration voltage of 40 kV and current of 40 mA was used. Samples were mounted on a Hampton Research CryoLoop. The 2-dimensional image plate data was converted to a conventional 1-dimensional powder pattern using Rigaku's 2DP Version 1.0 data conversion program. In this study the conversion range was from 2° to 40° at a 2θ step size of 0.01°. The data were analyzed using Bruker Analytical's DiffracPLUS Evaluation Package, EVA (V. 2009). XRPD patterns were established with the freshly prepared BMP formulations and the micronized blends, as well as the micronized and BMP individual APIs.

### E. Scanning electron microscopy

Scanning electron microscopy (SEM) was employed to evaluate the surface morphology of both micronized and BMP formulations. Prior to imaging, samples were loaded onto double sided carbon tape and coated with platinum/palladium targeted for 12 nm thickness using sputter coater 208 HR (Cressington Scientific Instruments, Watford, England). SEM images were captured using a SmartSEM® graphical user interface software in a Carl Zeiss Supra® 40VP (Carl Zeiss, Oberkochen, Germany) under high vacuum mode with an operating Electron High Tension (EHT) of 5 kV.

### F. Specific surface area analysis

Specific surface area (SSA), which is related to powder porosity, was measured using a Monosorb MS-22 rapid surface area analyzer (Quantachrome Instruments, Boynton Beach, Florida) with 30% nitrogen in helium as the adsorbate gas. Powder samples were degassed in a Thermoflow™ Degasser for at least 2 hours at 30 °C using nitrogen prior to analysis. The Monosorb utilizes a modified BET equation for extremely rapid, single-point determinations of surface area (P/P0 = 0.294).

### G. Fourier-transform infrared spectroscopy

Infrared spectra were acquired using Nicolet™ iS™50 FT-IR Spectrometer (Thermo Scientific, Waltham, MA) equipped with the Polaris™ long-life IR source (Thermo Scientific, Waltham, MA) and a DLaTGS-KBr detector. Each sample was dispersed in KBr using a mortar and pestle, and then a pellet was formed by applying pressure.

FTIR measurements were performed in the transmittance mode. The scanning range was from 700 cm⁻¹ to 3600 cm⁻¹, with a resolution of 4 cm⁻¹, 16 scans were recorded for each spectrum and the spectra were corrected against the background spectrum. All measurements were performed at room temperature.

### 2.8. Water Sorption

Water sorption profiles were determined for the BMP formulations using Dynamic Vapor Sorption (Surface Measurement Systems Ltd, London, UK). Consideration of the capacity (0.5 mL) of glass sample cells, 3 to 6 mg samples were loaded depending on particle density. As an initial step in all experiments, water absorbed during environmental exposure in storage or loading was removed by holding the samples at 0% relative humidity (RH) for 480 min or until the mass changed by less than 0.002% in dm/dt for 40 min. Each formulation was run for a complete sorption/desorption cycle between 0 and 90% relative humidity (RH) in steps of 10% RH at 25 °C. The instrument was run in dm/dt mode to decide when equilibrium was reached, as determined by a dm/dt less than 0.002% within an interval of 5 min. Sorption isotherms were calculated and plotted according to percent change in mass minus the initial dry formulation weight. The mass % sorption (desorption) was calculated by dividing the increase (decrease) in mass due to water sorption (desorption) by the mass of the dry BMP formulations after the initial equilibration at 0% RH, and multiplying by 100.

### H. Aerodynamic Particle Size Analysis

A Next Generation Pharmaceutical Impactor (NGI) (MSP Corp., Shoreview, MN) was used to determine the influence of DPI devices and to compare aerodynamic properties of BMP formulations and crystalline micronized blends. Around 2 mg powders were manually filled into size 3 HPMC capsules (Capsugel, Peapack, NJ) and aerosolized using a Miat monodose inhaler® DPI device, which was attached to the induction port by a molded silicone mouthpiece adapter. To characterize the performance of BMP formulations and micronized powders *in vitro* for animal study, powders were also filled into the insufflator chamber without capsule. Aerosols were produced over 2.7 s at an air flow rate of 91 L/min for Miat monodose inhaler® and 4 s at an air flow rate of 51 L/min for insufflator to achieve an inhalation volume of 4 L and a 4 kPa pressure drop across the device (Wang *et al.,* 2014; Watts *et al.,* 2013). Stage cut-off size diameters of both devices were calculated to be 6.48, 3.61, 2.30, 1.37, 0.76, 0.43, 0.26 and 0.26 µm stages 1 through 7 and micro-orifice collector (MOC) (Marple *et al.,* 2003). Before each run, NGI collection surfaces were coated with 1% (v/v) polysorbate 80 in ethanol, which is a coating material recommended by the European Pharmaceutical Aerosol Group (EPAG), to avoid bias caused by particle bounce and re-entrainment[41]. After aerosolization, all collection surfaces were rinsed with known volumes of mobile phase. Powders deposited in the capsule, device, adaptor, throat, pre-separator, and stages 1- MOC were extracted respectively. After passing through a 0.2 µm PTFE filter, the solutions were analyzed by HPLC for SX and MF content. A pre-separator was equipped for DPI devices, not for an insufflator. For each test, total emitted dose (TED) was defined as the percentage of API emitted from the inhaler with respect to the total loaded dose into the capsule. Geometric standard deviation (GSD) and mass median aerodynamic diameter (MMAD) were calculated according to the USP 32-NF 27 General Chapter 601 (DiNunzio *et al.,* 2008), based on the dose deposited on stages 1 - MOC. The fine particle fraction (FPF) was defined as the mass fraction of particles less than 5.0 µm with the emitted dose. A plot of cumulative percentage of mass less than stated aerodynamic diameter versus aerodynamic diameter (cut-off size for each stage) was built and fit to a 4 parameter logistic curve using Sigmaplot (Systat Software Inc., San Jose, CA).

### I. Chromatographic analysis

All rinsed surface collections from capsule, device, adaptor, throat, pre-separator and stages 1 - MOC were filtered and analyzed by high performance liquid chromatography (HPLC). HPLC was used to quantify the collected SX and MF. Samples were analyzed using a Dionex 3000 high performance liquid chromatography (HPLC) system equipped with a Phenomenex® reversed phase C₁₈ column 5 µm 150 mm × 4.60 mm. All tests were conducted at room temperature with a flow rate of 1.2 mL/min. The mobile phase consisted of 90/10 (v/v) methanol/water, pH 3.5 adjusted with phosphoric acid. The injection volume was 20 µL and the detection wavelength was 250 nm. Linearity test solutions were prepared from SX and MF stock solutions at seven-concentration levels ranging from 1 µg/mL to 250 µg/mL.

The SX is marketed as racemic mixture and no evidence indicates that the (S)-salmeterol has different effects compared to the corresponding (R)-salmeterol, thus chiral chromatographic analysis was employed to identify the chirality of BMP formulations. To determine the optical activity of SX in the inhaled powders, chiral chromatography was performed using a Dionex 3000 HPLC, and a Phenomenex® normal phase chiral column Lux Cellulose-4 (5 µm 250 × 4.60 mm). The stereochemical separation of (R)-salmeterol and (S)-salmeterol was accomplished using a mobile phase containing n-hexane/2-propanol (90:10, v/v) with 0.1% DEA. The analysis was carried out at ambient temperature using a flow rate 1.0 mL/min at 250 nm UV.

### J. In vivo pharmacokinetic studies

An animal study was approved by the University of Texas at Austin Institute of Animal Care and Use Committee (IACUC). Male and female Sprague Dawley rats with their jugular vein pre-catheterized and non-pre-catheterized (Charles River Laboratories International, Inc., Wilmington, MA) weighing between 250-300 g with an average weight of 270 g were used for the *in vivo* intubation and dry powder insufflation procedure. During one week of acclimation time, the rats were housed two per cage in a 12-h light/dark cycle with food and water available ad libitum. Catheters were flushed every 3 days before the study with 500 Units/mL heparinized normal saline. Animals were divided into two groups, and each group contained 12 non pre-catheterized rats for lung sample collection and 4 pre-catheterized rats for blood sample collection.

A DP-4M Dry insufflator™ device and AP-1 air pump and LS-2 small animal laryngoscope were used (Penn-Century, Inc., Wyndmoor, PA). BMP and micronized blends were weighed and loaded into the chamber of the insufflator (target SX dose = 450 µg/kg, target MF dose = 1980 µg/kg) before being attached to the adjustable-volume air pump. Each rat was anesthetized intraperitoneally with a 0.2 micron filtered mixture of ketamine HCl/xylazine HCl (80 mg/kg ketamine HCl, 6 mg/kg xylazine HCl), about 0.2-0.3 mL of the mixture was administered based on weight. After the rat was anesthetized, it was placed on its back at a 45-degree angle, and the incisors were secured with a small rubber band. The laryngoscope was used to visualize the trachea, then the insufflator device was inserted into the trachea and the air pump was attached to the insufflator device. The desired volume (2 mL of air per pump for rats) of air was dispensed from the attached air pump to force the powder from the chamber of the insufflator device through the PE tube, the metal-tipped cannula, and into the trachea of the rats (Morello *et al.,* 2009). All powders were actuated into the lungs using 4-7 pumps. The insufflator was weighed before and after powder filling and administration, to confirm the actual dose insufflated by mass.

Following insufflation, blood samples were withdrawn from a jugular vein catheter at time points of 5 min, 15 min, 30 min, 1 h, 2 h, 4 h, 8 h, 12 h and 24 h followed by injecting the equal volume of warm normal saline back into the rats and stored in a BD Vacutainer® blood collection tube. In each group, three rats were sacrificed at each time point (15 min, 1 h, 2 h, 6 h and 24 hours) for lung harvest. To investigate the amount of API deposited in the lower respiratory tract, a modified bronchoalveolar lavage (BAL) procedure was performed by washing respiratory tract twice with 3 mL sterile phosphate buffered saline (PBS) for collection of fluids prior to lung sample collection (Chougule *et al.,* 2007). Approximately 4-5 mL BAL was yielded. All bio samples were stored in -80 °C freezer until analyzed.

### K. Quantification of SX and MF Concentrations in the Lung tissue, BAL and Blood

SX and MF concentrations in the lung tissue, BAL and plasma were quantified by liquid chromatography mass spectrometry (LC/MS/MS). Lorcaserin (LOR) was employed as the internal standard. The LC/MS/MS system consisted of a Shimadzu CBM-20A Controller, two LC-20AD pumps, SIL-20AC autosampler, CTO-20AC column oven, and an AB Sciex API 4000-QTrap mass spectrometer with turbo ion spray. Chromatographic separation was achieved with an Ace Excel 3 Super C18 (3 × 75 mm, 3 µ) purchased from MacMod (Chadds Ford, PA) and was maintained at 40 °C during the chromatographic runs. Mobile phase A contained 0.1% formic acid in Millipore H₂O and mobile phase B contained 0.1% formic acid in acetonitrile. The flow rate of the mobile phase was 0.7 mL/min. SX and MF were eluted with a step gradient. The column was equilibrated with 60% mobile phase B. At 2.5 minutes after injection, the system was switched to 95% mobile phase B. At 6 min, the system was switched back to 60% mobile phase B in preparation for the next injection. The following Q1/Q3 transitions were monitored: m/z 416.1 → 232.0 for SX, m/z 521.9 → 355.2 for MF, and m/z 196.0 → 129.1 for LOR (IS). The underlined transitions were used for quantification.

Quantification of SX and MF in plasma: SX/MF was quantified in plasma (EDTA). Briefly, 100 µL of calibrator and unknown plasma samples were mixed with 10 µL of LOR working stock solution (internal standard) and 300 µL of Mobile Phase B (0.1% formic acid in acetonitrile). The samples were vortexed vigorously for 2 min, and then centrifuged at 3200 g for 20 min at 23 °C. Supernatant was transferred to 1.5 mL microfilterfuge tubes, centrifuged, and then 50 µL of the final extracts were injected into the LC/MS/MS. The ratio of the peak area of SX and MF to that of the internal standard LOR (response ratio) for each unknown sample was compared against a linear regression of calibrator response ratios at 0, 0.5, 1, 10, 50, 100, 500, 1000 ng/mL to quantify SX and MF. The concentration of each analyte was expressed as ng/mL plasma.

Quantification of SX and MF in lung tissue: the lung's weight was 1.6 grams on average. Briefly, calibrator, control, and unknown tissue samples were mixed with a 10 volumes of mobile phase B and homogenized with a tissue homogenizer. Samples were centrifuged at 3200 g for 20 min and then 100 µL of supernatant were transferred to 1.5 mL microfilterfuge tubes and spiked with 10 µL of LOR working stock solution. The samples were vortexed vigorously for 2 min, transferred to 1.5 mL microfilterfuge tubes, centrifuged, and then 10 µL of the final extracts were injected into the LC/MS/MS. The ratio of the peak area of SX and MF to that of the internal standard lorcaserin (response ratio) for each unknown sample was compared against a linear regression of calibrator response ratios at 0, 10, 100, 1000, 2500, 5000, 7500, 10000 ng/mL to quantify SX and MF. The concentration of SX and MF was normalized to protein content and expressed as µg/g of tissue (parts per million).

Quantification of SX and MF in BAL: 100 µL of calibrator, control, and unknown samples were mixed by vortexing with 300 µL of Mobile Phase B. After vortexing the samples were transferred to 1.5 ml microfilterfuge tubes and then centrifuged at 3200 g for 15 min at 23 °C. Filtrates were transferred to autosampler tubes and 10 µL of the final extracts were injected into the LC/MS/MS. The peak area of SX and MF for each unknown sample was compared against a linear regression of calibrator peak areas at 0, 0.5, 1.0, 5, 10, 50, 100, 1000, 5000, 10000 ng/mL to quantify SX and MF. The concentration of SX and MF was expressed as ng/mL BAL.

### Example 4 - Results for SX and MF Combination

### A. Differential scanning calorimetry

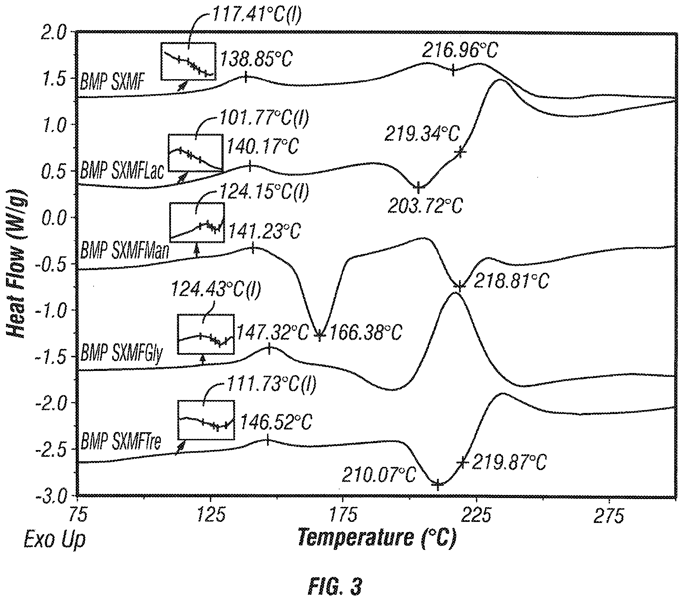

The results shown in FIG. 3 indicate that BMP formulations exhibited no melting endotherms, however a single glass transition (T_{g}) in the temperature range of 101.77 °C to 124.43 °C was observed by modulated DSC, which was followed by a single recrystallization peak in the temperature range of 138.85 °C to 147.32°C, respectively.

### B. X-Ray Powder Diffraction

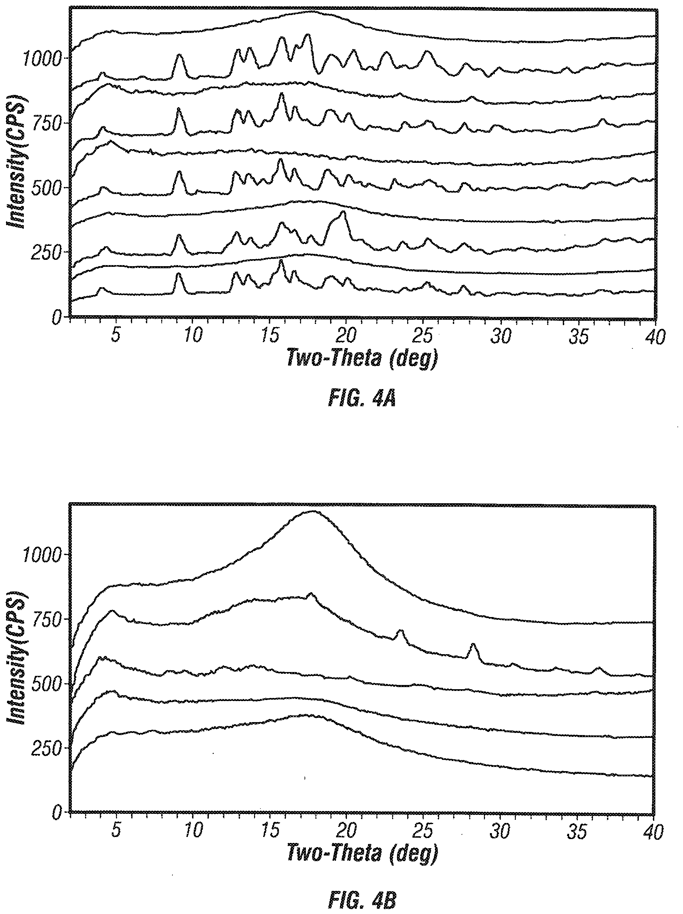

Absence of crystallinity was confirmed in FIG. 4A by the presence of a halo pattern in the diffractogram for the fixed dose combinations BMP SXMF, BMP SXMFLac and BMP SXMFTre after lyophilization. However, excipients in the fixed dose combination drug mixtures exhibited different crystalline properties as evidenced by XRPD. Gly and Man peaks were observed for BMP SXMFGly and BMP SXMFMan. The physical blends of the micronized drugs exhibited characteristic crystal peaks for SX and MF, respectively.

Stability results shown in FIG. 4B conducted on the fixed dose combinations prepared by TFF at 25 °C/30% RH for 6 months exhibited no changes in XRPD peak intensity of BMP SXMF and BMP SXMFTre over 6 months, and the powders remained amorphous. In contrast, XRPD diffraction patterns showed a small crystalline peak corresponding to the SX (4.21 2θ) was observed for BMP SXMFLac, BMP SXMFMan and BMP SXMFGly over the same time period.

### C. Scanning electron microscopy and specific surface area analysis

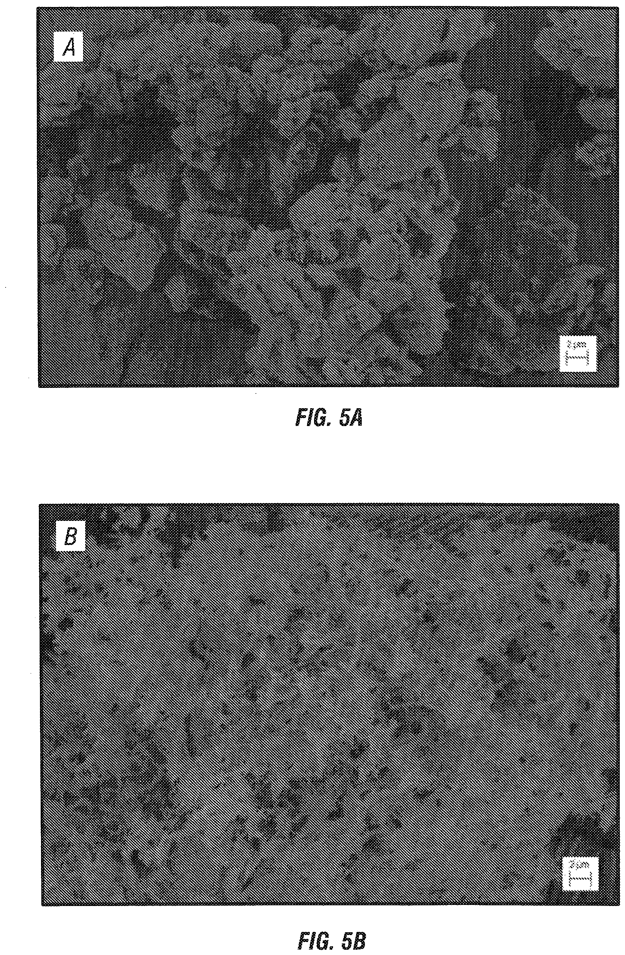

As exemplified in FIGS. 5A & 5B, the BMP fixed dose combinations exhibited porous microparticulate aggregates loosely connected to form a sponge-like structure with adjacent aggregates, which appeared homogeneous and brittle. In contrast, micronized drug physical blends were not homogeneous.

The results of BET surface area measurements are shown in Table 3. The specific surface area of micronized SXMF was found to be 0.89 m²/g. Generally, the specific surface area of BMP fixed dose combinations for co-deposition varied from around 26.87 to 35.61 m²/g. BMP SXMFLac and BMP SXMFTre showed larger surface areas as compared to the other BMP formulations. However, there was no significant difference between the values of BMP formulations.

**Table 3: Specific surface area (SSA) of micronized SXMF, BMP SXMF, BMP SXMFLac, BMP SXMFMan, BMP SXMFGly and BMP SXMFTre. Values are expressed as mean ± SD (n=3).**

| **Formulation** | **SSA (m²/g)** |
|---|---|
| Micronized SXMF | 0.89±0.01 |
| BMP SXMF | 28.18±1.038 |

| **Formulation** | **SSA (m²g)** |
|---|---|
| BMP SXMFLac | 35.61±1.682 |
| BMP SXMFMan | 29.87±0.588 |
| BMP SXMFGly | 26.87±1.255 |
| BMP SXMFTre | 32.41±0.456 |

### D. Fourier Transform Infrared Spectroscopy

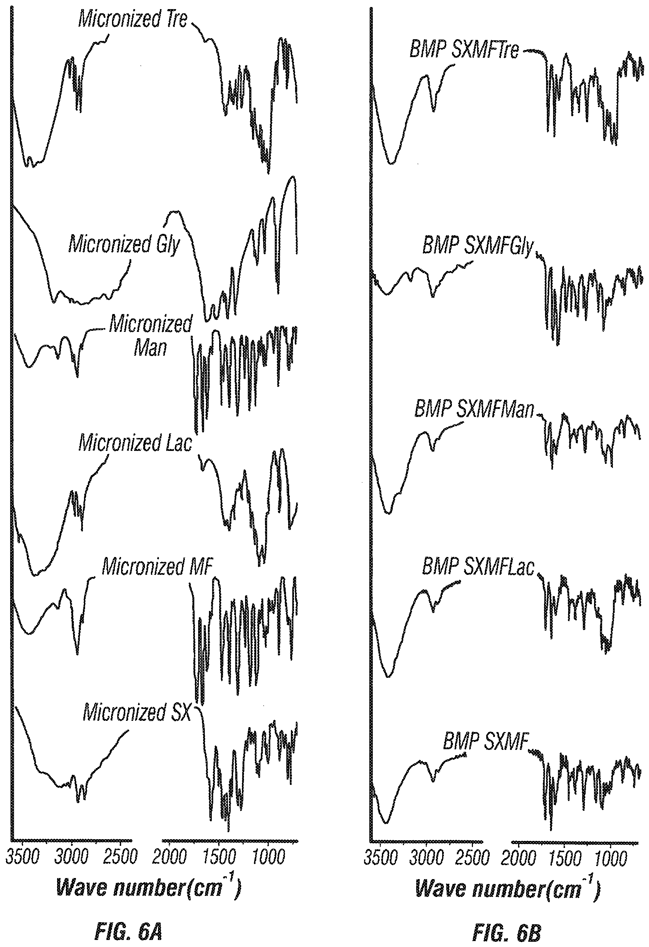

As shown in FIGS. 6A & 6B, Fourier transform infrared spectroscopy (FTIR) spectrum for individual components and TFF processed powders were investigated and examined. The peak of -C=O stretching at 1724 cm⁻¹ could be observed for all BMP, also including the single SX and MF. The strong peak located in the region of 3200 cm⁻¹ to 3700 cm⁻¹ could correspond to the -OH or -NH, which was observed in all single materials. Single SX showed strong peaks at 3278 cm⁻¹ (shown in FIG. 6A), which was attributed to -NH stretching of the secondary amide. The -C-N stretching for SX was confirmed by presence of relatively sharp peak at 1268 cm⁻¹. In the carbonyl region, the individual crystalline forms exhibited broad peaks compared to fixed dose combinations. No band shift was observed at the -C=O stretching peak at 1724 cm⁻¹ in all fixed dose combinations as compared to crystalline MF. Strong and wide -NH and -OH peaks in the region of 3200 cm⁻¹ and 3400 cm⁻¹ were seen for the single materials, especially for the excipients. Overall, the wave numbers of either - C=O or -NH and -OH did not shift for fixed dose combinations after TFF process. This lack of shifting indicates no evidence of H-bonding.

### E. Water Sorption

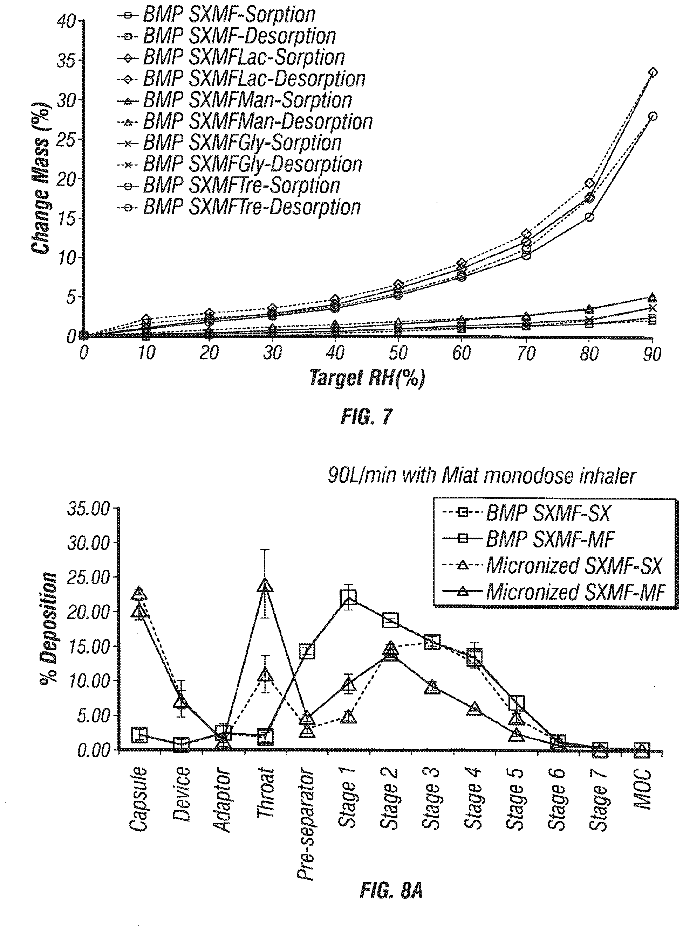

FIG. 7 showed the results of BMP fixed dose combinations for the sorption and desorption isotherms determined after one cycle by DVS. Slow moisture uptake was observed from BMP SXMFLac and BMP SXMFTre during sorption testing. The time required to a complete the sorption/desorption cycle was 22 h and 19 h, respectively, since slow water absorption (and corresponding slow baseline stabilization) meant long cycle time compared to BMP SXMFMan (12 h), BMP SXMFGly (11.5 h) and BMP SXMF (11 h). The moisture uptake as measured by weight gain (%) was BMP SXMFLac (∼ 35%), BMP SXMFTre (∼ 30%), BMP SXMFMan (∼ 5%), BMP SXMFGly (∼ 4%) and BMP SXMF (∼ 2%). This suggests that the BMP SXMFLac and BMP SXMFTre were more hydroscopic than the other compositions.

### F. In vitro aerosol performance

The NGI dispersion data for the fixed dose BMP formulations and micronized API blends were plotted and shown in Table 4. The aerosol properties of all fixed dose combinations administered with the Miat monodose inhaler® revealed high FPF (38%-57%) and small MMAD (around 4 µm).

**Table 4: Aerodynamic properties of SX and MF for co-drug deposition BMP and crystalline micronized powders under 90 L/min with Miat monodose inhaler®. Values are expressed as mean ± SD (n=3).**

| **Formulation** | | **FPF (% of deliv.)** | **FPF (% of loaded)** | **MMAD (µm)** | **GSD (µm)** | **TED (%)** |
|---|---|---|---|---|---|---|
| BMP SXMF | SX | 55.52±4.78 | 52.90±4.18 | 3.58±0.32 | 3.55±1.12 | 95.31±1.13 |
| | MF | 52.08±4.40 | 48.91±4.16 | 3.69±0.26 | 3.33±0.63 | 93.91±1.27 |
| BMP SXMFLac | SX | 42.13±1.46 | 40.31±0.76 | 3.68±0.22 | 2.39±1.21 | 95.72±2.28 |
| | MF | 40.90±1.26 | 38.64±0.97 | 3.97±0.35 | 3.56±0.60 | 94.50±1.64 |
| BMP SXMFMan | SX | 44.60±4.09 | 42.74±3.22 | 4.45±0.46 | 3.67±1.29 | 95.92±2.07 |
| | MF | 43.91±3.37 | 41.06±3.47 | 4.43±0.36 | 3.86±1.53 | 93.49±1.70 |
| BMP SXMFGly | SX | 56.45±7.38 | 53.57±5.87 | 3.62±0.75 | 2.66±0.36 | 95.14±4.27 |
| | MF | 53.38±6.55 | 49.98±5.02 | 3.74±0.68 | 2.58±0.25 | 93.86±3.99 |
| BMP SXMFTre | SX | 42.18±3.37 | 39.76±1.27 | 4.09±0.57 | 2.64±0.55 | 94.55±5.43 |
| | MF | 37.85±5.74 | 35.93±4.77 | 4.10±0.48 | 2.61±0.46 | 95.13±3.24 |
| Micronized SXMF | SX | 59.51±7.07 | 40.98±6.77 | 3.30±0.62 | 1.91±0.06 | 68.59±3.57 |
| | MF | 34.29±5.78 | 24.50±4.91 | 4.45±0.96 | 2.01±0.02 | 71.18±3.12 |
| Micronized SXMFLac | SX | 56.15±9.58 | 41.39±8.60 | 3.65±0.58 | 1.00±0.01 | 73.88±1.14 |
| | MF | 27.27±6.01 | 21.09±4.37 | 4.84±0.81 | 1.90±0.04 | 77.49±3.46 |
| Micronized SXMFMan | SX | 57.52±7.91 | 44.74±6.82 | 3.41±0.65 | 1.94±0.07 | 77.67±1.66 |
| | MF | 30.07±6.20 | 23.84±5.05 | 4.84±0.92 | 1.94±0.02 | 79.20±0.57 |
| Micronized SXMFGly | SX | 59.70±9.63 | 47.96±7.58 | 3.39±0.67 | 1.88±0.09 | 80.37±0.34 |
| | MF | 32.73±8.49 | 26.58±5.84 | 4.71±L1.02 | 1.89±0.06 | 81.86±3.86 |
| Micronized SXMFTre | SX | 60.23±3.50 | 47.11±2.71 | 2.91±0.29 | 1.91±0.05 | 78.55±8.66 |
| | MF | 19.81±5.86 | 16.54±7.14 | 4.29±0.37 | 1.92±0.06 | 81.54±9.34 |

However, the aerosol properties of SX corresponded with MF for fixed dose BMP formulations. The FPF of micronized blend SXMF was 59.51±7.07% for SX versus 34.29±5.78% for MF, while the FPF of BMP SXMF was 55.52±4.78% for SX versus 52.08±4.40% for MF, which were similar. Surprisingly, the MMADs for both formulations displayed similar values loading in the range of 3-5 µm. However, the MMAD of two APIs were close for fixed dose combination. Conversely, the micronized blends of SX and MF exhibited different MMADs. In addition, TEDs of two APIs for BMP formulations were approximately above 93% and the TED for micronized blends was below 82%.

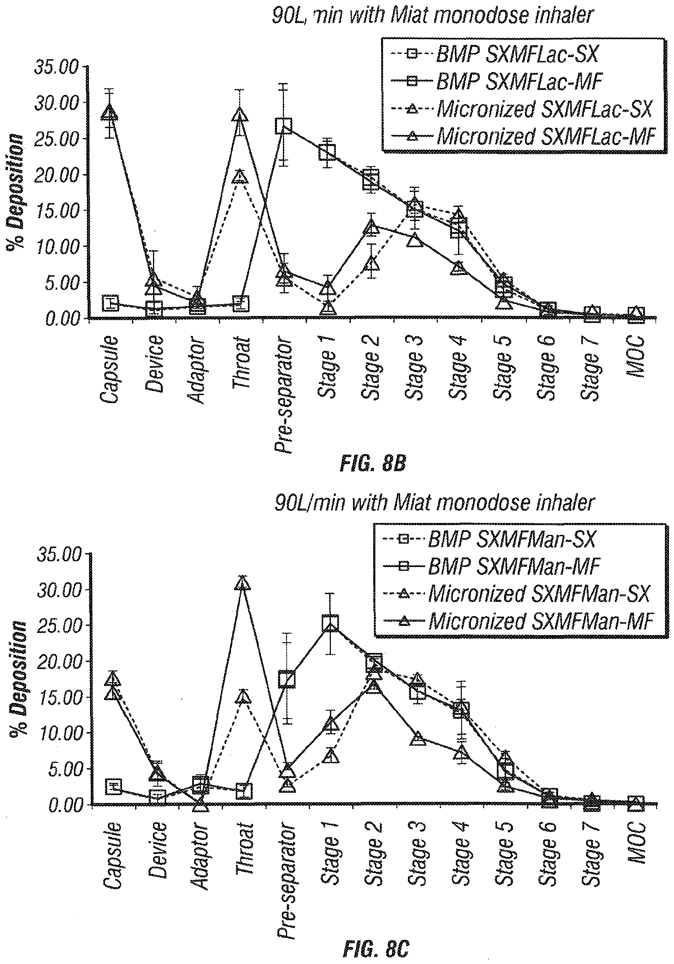

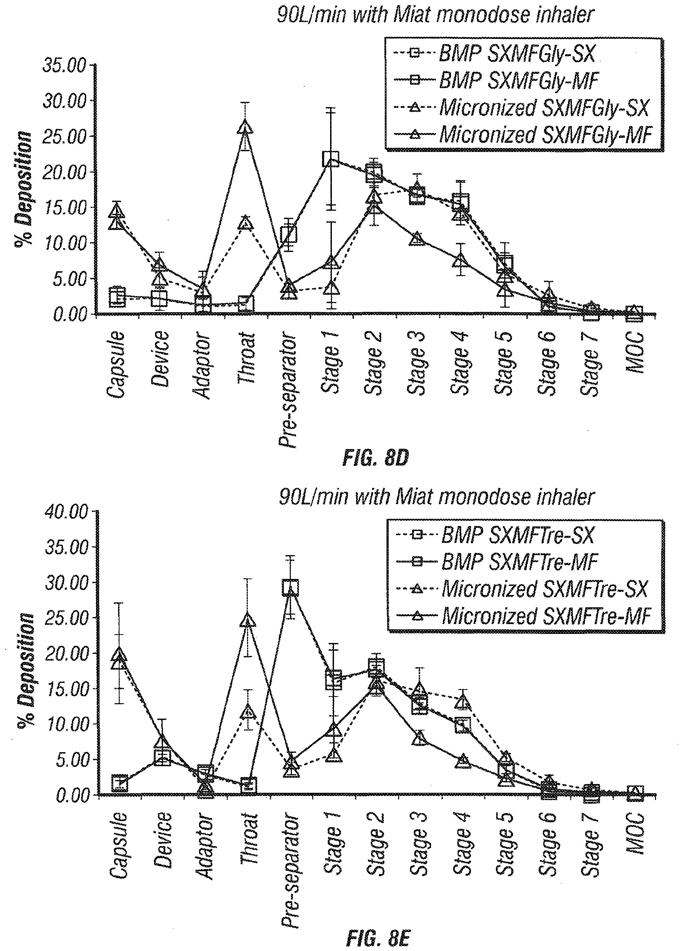

Inspection of the NGI data in FIGS. 8A-8E showed that, the deposition percentages in different stages between SX and MF were similar for BMP formulations. BMP fixed dose combinations for co-deposition produced dose proportional aerosol of SX with equivalent performance of the other component MF by percentage deposition. Micronized blends exhibited non-uniformed distribution between SX and MF with the same components consisted as shown in FIGS. 8A-8E. Moreover, the micronized blends were deposited primarily in the capsule and throat, while BMP formulations were deposited primarily in the pre-separator and stage 1. BMP SXMF and BMP SXMFGly exhibited higher FPF and lower MMAD.

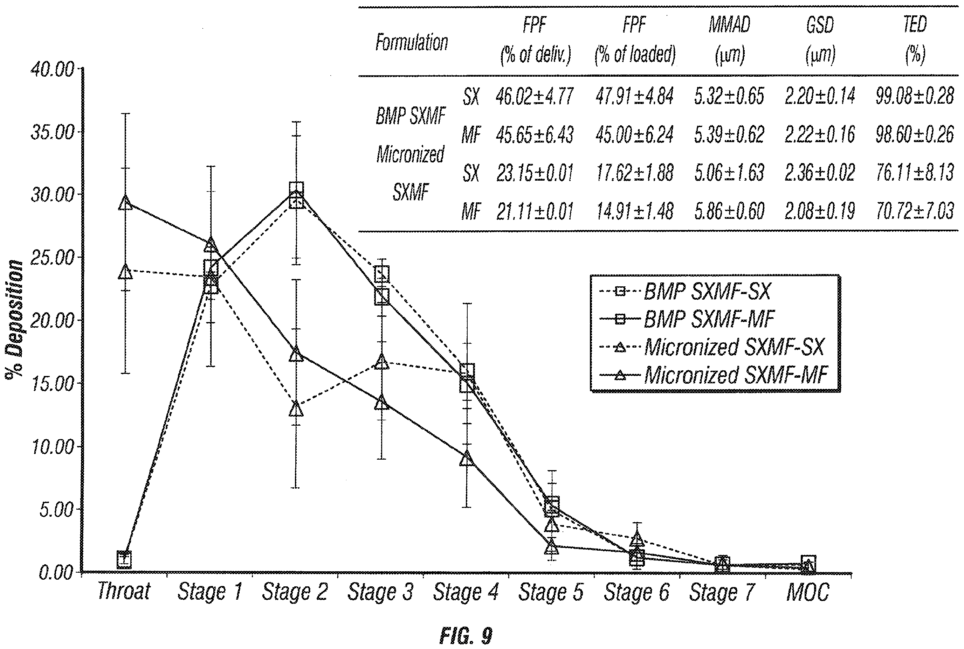

The aerodynamic properties of BMP SXMF and micronized SXMF performed by insufflator using 51 L/min air flow rate through the NGI apparatus were reported in FIG. 9. The FPFs of BMP SXMF were 46.02±4.77% and 45.65±6.43% for SX and MF, and the FPFs of micronized SXMF were 23.15±0.01% and 21.11±0.01%. Though the values of MMAD and GSD were close for both formulations, TEDs of BMP SXMF were almost 99%.

### G. Chromatography

Linearity test solutions of SX and MF were conducted with seven concentrations in the range of 1 µg/mL to 250 µg/mL (r>0.99). The recovery rate (%) was between 85%-105%, which was considered reliable, otherwise the test was repeated.

In chiral chromatography, two peaks were observed for all fixed dose combinations and the peak areas were similar indicating that the chirality was not changed after lyophilization, and the retention time of SX chirality was approximately 18 minutes and 21 minutes.

### Example 4 - Discussion for SX and MF Combination Therapy

The combo-BMP of SX and MF (in mass ratio of 5:22) was produced by TFF to enable co-deposition of two APIs in consistent ratio throughout the airway. Jet milling was used in order to prepare micronized reference blends for comparison. Lactose (Lac), mannitol (Man), glycine (Gly) and trehalose (Tre) were employed to prepare SX and MF BMP formulations. Unlike coarse blended DPI products which use excipients as carrier particles, the pharmaceutical excipient in BMP form the matrix structure and stabilize amorphous API. Lactose, mannitol and glycine are approved by the U.S. Food and Drug Administration (FDA) as excipients for inhalation and are used in DPIs in differing amounts (e.g., up to 25 mg of lactose (Watts *et al.,* 2013), 0.051% of mannitol and 0.01% of glycine). Trehalose is widely used in pharmaceutical formulations as a lyoprotectant because of its relatively high T_{g} and low tendency to crystallize (Simperler *et al.,* 2006). On the molecular level, SX, MF and the excipients used herein have functional groups that provide hydrogen bond donors and acceptors, such as carboxylic acid, amides, and alcohols, which have been shown to improve the stability of amorphous systems when used in combination (Jeffrey G.A., 1997).

To formulate BMP combinations for co-deposition, it is desirable for SX and MF to be thermodynamically miscible after TFF processing. The appearance of a single T_{g} and recrystallization peak as well as absence of melting peak for two APIs of BMP formulations confirmed the formation of API - API homogeneous amorphous phase, where one API was dissolved in the other API or the excipients. Based on DSC thermograms, it is suggested that BMP SXMF was transferred into co-amorphous state. Thermal properties of excipients were not clear enough to be estimated, because of API degradation. However, the morphological state of individual components is clearer in XRPD studies. XRPD patterns of BMP indicate amorphous API in agreement with DSC thermograms. The crystallography of excipients is also clearly observed. As seen in FIG. 4A, the absence of the characteristic SX and MF crystalline peaks and the appearance of typical halo diffraction patterns in BMP formulations suggested an amorphous morphology of SX and MF. These results indicated amorphous binary (BMP SXMF) or ternary (BMP SXMFLac or BMP SXMFTre) solid dispersions were formed by the introduction of high T_{g} components, that the T_{g} of MF, Lac and Tre was approximately 150°C, 116°C (Craig *et al.,* 2000) and 94°C (Simperler *et al.,* 2006). Though SX and MF were amorphous in BMP formulations containing crystalline Gly and Man, crystallization of API may occur over time due to nucleation and crystal seeding (Sun *et al.,* 2012).

Co-amorphous solids were produced to increase the solubility, stability and bioavailability, thus amorphous APIs must resist their thermodynamic tendency to crystallize in order to maintain these advantages. The increased stability of amorphous systems was generally explained by the increased T_{g}, which may reduce the molecular mobility required for crystallization at certain storage temperatures (Janssens and Van den Mooter, 2009). In this study, the BMP SXMF and BMP SXMFTre still remained in good co-amorphous conditions after stored at 25 °C/30% RH for 6 months. However, a small SX crystalline peak at 4.21 2θ was observed for BMP SXMFLac, BMP SXMFMan and BMP SXMFGly over the same time period.

Without wishing to be bound by any theory, it is believed that the specific surface area and morphology of API particles can significantly influence the API release characteristics through both kinetic and thermodynamic effects (DiNunzio *et al.,* 2008). The difference in size, density, specific surface area and morphology can be attributed to the process of particle formation (Ali and Lamprecht, 2014). Unlike irregular and cohesive micronized blends, an advantage of BMP formulations was the porous and fragile structure facilitates the potential for rapid dissolution of the API in pulmonary fluid (Janssens and Van den Mooter, 2009). The SSA of BMP formulations were greatly increased compared to the micronized formulations due to the porous matrix structure. All TFF processed powders exhibited very low bulk densities and were expected to aerosolize readily.

Moisture sorption plays a key role in the aerosol characteristics of BMP formulations. It has been reported that respirable BMPs prepared with lactose as excipient by TFF were susceptible to moisture induced matrix collapse and hygroscopicity; in contrast, the aerosolization properties of BMPs were not influenced by high humidity when processed with mannitol (Watts *et al.,* 2013). Without wishing to be bound by any theory, it is believed that hygroscopic excipients may increase the risk of instability in co-amorphous BMP formulations (based on the stability study), while BMP formulations processed with non-hygroscopic excipient or no excipient were more robust (as revealed from *in vitro* aerosol performance).

Fourier transform infrared spectroscopy (FTIR) measurements were carried out to gain insight into possible molecular level interactions, between SX, MF and excipients in BMP formulations. Crystal single ingredients were analyzed in order to make an easy comparison in this study (Heinz *et al.,* 2009). It was impossible to determine if there was shift because of hydrogen bonding interactions in the region of 3000 cm⁻¹ and 3500 cm⁻¹ corresponding to the -OH stretching, due to the effects of thermal excitation on these vibrational modes for excipients.

Though the strong -NH and -OH peaks in the region of 3200 cm⁻¹ and 3400 cm⁻¹ for the single materials changed shapes, no significant peak shifts could be detected for either -C=O or -NH and -OH in all BMP fixed dose combinations. Thus, wishing to be bound by any theory, it is believed that no interaction occurred. Though hydrogen-bond interactions are usually observed in co-amorphous formulations especially in the formulations composed of materials structured with potential hydrogen receptors and donors, it was not inevitable for this to occur and not necessary for improving their physiochemical properties (Dengale *et al.,* 2014; Lobmann *et al.,* 2012).

Aerodynamic performance is one factor in measuring the performance for inhaled dry powder formulations as it is correlated to lung deposition (Ali and Lamprecht, 2014). Though the BMP formulations produced large geometric diameters, low particle density allows for sufficiently low aerodynamic diameters for effective lung deposition (Carvalho *et al.,* 2014; Watts *et al.,* 2013). The Miat monodose inhaler® was employed herein, which provided the necessary shear force to break up the brittle matrices into respirable particles. In addition, powders delivered from a powder insufflator were characterized in the preparation for the *in vivo* study.

More BMP formulation emptied from the capsule and adaptor than the micronized blends, as demonstrated by the higher TED. The FPF (% of delivered) of SX in BMP formulations was improved by 25% compared to a previously investigated SX combination formulation prepared by anti-solvent precipitation(Murnane *et al.,* 2009). For the monotherapy MF Twisthaler, it has been reported that the FPF of 200 µg strength is 27.9% (Berger and Berger, 2013), thus the FPF of MF-DPI was increased significantly as well in this study. The data presented in Table 4 showed that the aerosol properties of SX and MF for BMP formulations were similar, suggesting that both APIs were co-deposited after inhaled *in vitro.* Conversely, micronized SX and MF delivered in a combination powder blend demonstrated different FPFs and MMADs.

Aerodynamic particle size distribution within the NGI, shown in FIG. 8A-8E suggested that APIs of BMP formulations were delivered to all stages with highly consistent, while micronized blends resulted in variance. It has been reported that after administration of SX and a corticosteroid fluticasone propionate from a single inhaler, co-location on the cellular level offers a potential for increased clinical efficacy (Nelson *et al.,* 2003). Thus, it may be that this fixed dose combination offers increased opportunity for synergistic interaction to occur than the same dose of two separate inhalers.

Powders formulated with non-hydroscopic excipients or no excipient showed elevated FPFs compared to hydroscopic powders, for example BMP SXMF versus BMP SXMFLac as shown in Table 4. Based on the comparison of deposited percentage for SX and MF, without wishing to be bound by any theory, it is believed that reduced surface cohesion of BMP formulations lead to low capsule retention which was normally caused by van der Waals, capillary, and electrostatic forces in traditional formulations (Watts *et al.,* 2013). Though FPFs as a percentage of loaded dose were around 38%-57% for all two TFF formulated APIs, further optimization of formulation/device is needed to reduce upper airway (stage 1 and 2) deposition in these formulations. BMP SXMF powder was chosen for *in vivo* study due to its stability and good aerodynamic performance, meanwhile crystalline micronized SXMF blend was employed as reference. Moreover, the *in vitro* aerosol performance study suggested that insufflator could produce fine aerosol particles for animal study with both formulations.

The fixed dose combination BMP SXMF exhibited increased deposited amount of both APIs in lung tissue and increased bioavailability significantly in lung and blood. Further formulation approaches could be considered to decrease the extent of MF systemic absorption. Since long-acting β2-agonists (LABA) have the ability to prime glucocorticosteroid receptors and improve the activity of inhaled corticosteroids, the fixed dose combination BMP SXMF had potential better pharmacodynamics than traditional crystalline micronized SXMF blends.

### REFERENCES

The following references provide exemplary procedural or other details supplementary to those set forth herein.
U.S. Patent No. 5,725, 871
U.S. Patent No. 5,756,468
U.S. Patent No. 5,780,045
U.S. Patent No. 6,440,101
U.S. Patent No. 8,579,855
U.S. Patent Application No. 2010/0221343
PCT Patent Application Publication No. WO 2009/125986
Alhalaweh et al., Theophylline Cocrystals Prepared by Spray Drying: Physicochemical Properties and Aerosolization Performance. AAPS PharmSciTech. 2013 Jan 8;14(1):265-76.
Ali, M.E. and Lamprecht, A., Spray freeze drying for dry powder inhalation of nanoparticles, European journal of pharmaceutics and biopharmaceutics: official journal of Arbeitsgemeinschaft fur Pharmazeutische Verfahrenstechnik e.V, 87 (2014) 510-517, 2014.
Berger, R. and Berger, W.E., Particle size and small airway effects of mometasone furoate delivered by dry powder inhaler, in: Allergy and Asthma Proceedings, OceanSide Publications, Inc, pp. 52-58, 2013.
Carvalho et al., Characterization and pharmacokinetic analysis of crystalline versus amorphous rapamycin dry powder via pulmonary administration in rats, European journal of pharmaceutics and biopharmaceutics : official journal of Arbeitsgemeinschaft fur Pharmazeutische Verfahrenstechnik e.V, 88:136-147, 2014.
Chougule et al., Nano-liposomal dry powder inhaler of tacrolimus: preparation, characterization, and pulmonary pharmacokinetics, International journal of nanomedicine, 2:675-688, 2007.
Chowdhury, B.A. and Pan, G. Dal, The FDA and safe use of long-acting beta-agonists in the treatment of asthma, The New England journal of medicine, 362:1169-1171, 2010.
Craig et al., An evaluation of the use of modulated temperature DSC as a means of assessing the relaxation behaviour of amorphous lactose, Pharmaceutical research, 17:696-700, 2000.
Dengale et al., Preparation and characterization of co-amorphous Ritonavir-Indomethacin systems by solvent evaporation technique: improved dissolution behavior and physical stability without evidence of intermolecular interactions, European journal of pharmaceutical sciences : official journal of the European Federation for Pharmaceutical Sciences, 62:57-64, 2014.
DiNunzio et al., Amorphous compositions using concentration enhancing polymers for improved bioavailability of itraconazole, Molecular Pharmaceutics, 5: 968-980, 2008.
Evora et al., Pyrazinamide-diflunisal: a new dual-drug co-crystal, Crystal Growth & Design, 11:4780-4788, 2011.
French et al., The influence of formulation on emission, deaggregation and deposition of dry powders for inhalation. J Aerosol Sci. 1996 Jul;27(5):769-83.
Greening et al., Added salmeterol versus higher-dose corticosteroid in asthma patients with symptoms on existing inhaled corticosteroid. Allen & Hanburys Limited UK Study Group, Lancet, 344:219-224, 1994.
Heinz et al., Understanding the solid-state forms of fenofibrate-a spectroscopic and computational study, European journal of pharmaceutics and biopharmaceutics : official journal of Arbeitsgemeinschaft fur Pharmazeutische Verfahrenstechnik e.V, 71:100-108, 2009.
Hickey et al., Physical characterization of component particles included in dry powder inhalers. I. Strategy review and static characteristics. J Pharm Sci. 96(5):1282-301, 2007.
Janssens, S. and Van den Mooter, G, Review: physical chemistry of solid dispersions, The Journal of Pharmacy and Pharmacology, 61:1571-1586, 2009.
Jeffrey, G.A. An introduction to hydrogen bonding, Oxford university press New York, 1997.
Jouyban-Gharamaleki, et al., Solubility prediction of salmeterol xinafoate in water-dioxane mixtures, International Journal of Pharmaceutics, 216:33-41, 2001.
Liao, X. and Wiedmann, T.S., Solubilization of cationic drugs in lung surfactant, Pharmaceutical Research, 20:1858-1863, 2003.
Lobmann et al., A theoretical and spectroscopic study of co-amorphous naproxen and indomethacin, International journal of pharmaceutics, 453:80-87, 2013.
Lobmann et al., Co-amorphous simvastatin and glipizide combinations show improved physical stability without evidence of intermolecular interactions, European journal of pharmaceutics and biopharmaceutics : official journal of Arbeitsgemeinschaft fur Pharmazeutische Verfahrenstechnik e.V, 81:159-169, 2012.
M. JP, Practices of coating collection surfaces of cascade impactors: a survey of members of the european pharmaceutical aerosol group (EPAG), Drug Deliv Lung, 14 (2003) 4.
Mapel et al., The role of combination inhaled corticosteroid/long-acting beta-agonist therapy in COPD management, Primary care respiratory journal : journal of the General Practice Airways Group, 19:93-103, 2010.
Marple et al., Next generation pharmaceutical impactor (a new impactor for pharmaceutical inhaler testing). Part II: Archival calibration, Journal of aerosol medicine : the official journal of the International Society for Aerosols in Medicine, 16:301-324, 2003.
Morello et al., Dry-powder pulmonary insufflation in the mouse for application to vaccine or drug studies, Tuberculosis, 89:371-377, 2009.
Murnane et al., Dry powder formulations for inhalation of fluticasone propionate and salmeterol xinafoate microcrystals, Journal of Pharmaceutical Sciences, 98:503-515, 2009.
Nelson et al., Enhanced synergy between fluticasone propionate and salmeterol inhaled from a single inhaler versus separate inhalers, The Journal of Allergy and Clinical Immunology, 112:29-36, 2003.
Overhoff et al., Novel ultra-rapid freezing particle engineering process for enhancement of dissolution rates of poorly water-soluble drugs. Eur J Pharm Biopharm., 2007 Jan, 65(1):57-67.
Parikh et al., Improved localized lung delivery using smart combination respiratory medicines. Eur Respiratory Disease 8(1):40-45,2012.
Patil, J.S. and Sarasija, S., Pulmonary drug delivery strategies: A concise, systematic review, Lung India: official organ of Indian Chest Society, 29:44-49, 2012.
Qian, et al., Drug-polymer solubility and miscibility: Stability consideration and practical challenges in amorphous solid dispersion development, Journal of Pharmaceutical Sciences, 99:2941-2947, 2010.
Rasenack N and Muller BW, Micron-size drug particles: Common and novel micronization techniques. Pharm Dev Technol. 9(1):1-13, 2004.
Shayanfar et al., Coamorphous atorvastatin calcium to improve its physicochemical and pharmacokinetic properties, Journal of pharmacy & pharmaceutical sciences : a publication of the Canadian Society for Pharmaceutical Sciences, Societe canadienne des sciences pharmaceutiques, 16:577-587, 2013.
Silvasti et al., a novel multidose powder inhaler --- Comparison with metered dose inhaler, Drug Today, 32:11, 1996.
Simperler et al., Glass transition temperature of glucose, sucrose, and trehalose: an experimental and in silico study, The Journal of Physical Chemistry. B, 110:19678-19684, 2006.
Sin and Man, Do chronic inhaled steroids alone or in combination with a bronchodilator prolong life in chronic obstructive pulmonary disease patients? Curr Opin Pulm Med. 2007 Mar;13(2):90-7.
Sowa et al., A 1: 1 pharmaceutical cocrystal of myricetin in combination with uncommon piracetam conformer: X-ray single crystal analysis and mechanochemical synthesis, Journal of Molecular Structure, 1058:114-121, 2014.
Sun et al., Stability of amorphous pharmaceutical solids: crystal growth mechanisms and effect of polymer additives, The AAPS Journal, 14:380-388, 2012.
Tajber et al., Spray drying of budesonide, formoterol fumarate and their composites-I. Physicochemical characterisation. Int J Pharm. 2009 Feb 9;367(1-2):79-85.
Takenaga et al., J.Control Release, 52(1-2):81-87, 1998.
Trivedi et al., A Rapid, Stability-Indicating RP-HPLC Method for the Simultaneous Determination of Formoterol Fumarate, Tiotropium Bromide, and Ciclesonide in a Pulmonary Drug Product. Sci Pharm. 2012 Sep;80(3):591-603.
Vehring et al., Cosuspensions of Microcrystals and Engineered Microparticles for Uniform and Efficient Delivery of Respiratory Therapeutics from Pressurized Metered Dose Inhalers. Langmuir. 2012 Oct 23;28(42):15015-23.
Velaga et al., Supercritical fluids crystallization of budesonide and flunisolide. Pharm Res. 2002 Oct;19(10):1564-71.
Wang et al., In vitro and in vivo performance of dry powder inhalation formulations: comparison of particles prepared by thin film freezing and micronization, AAPS PharmSciTech, 15: 981-993, 2014.
Watts et al., "Respirable Low-Density Microparticles Formed In Situ from Aerosolized Brittle Matrices, Pharmaceutical Research, 30(3):813-825, 2013.
Weers et al., Pulmonary Formulations: What Remains to be Done? J Aerosol Med Pulm Drug Deliv. 2010 Dec;23:S5-S23.
Weers J and Tarara T, The PulmoSphere™ platform for pulmonary drug delivery. Therapeutic delivery 5:277-295, 2014.
Williamson et al., Paradoxical trough effects of triple therapy with budesonide/formoterol and tiotropium bromide on pulmonary function outcomes in COPD. CHEST Journal; 138:595-604, 2010.
Yang et al., Pharmaceutical Cryogenic Technologies. In Formulating Poorly Water Soluble Drugs. Springer; 443-500, 2012.
Zeng et al., The role of fine particle lactose on the dispersion and deaggregation of salbutamol sulphate in an air stream in vitro. Int J Pharm. 1998 Dec 30;176(1):99-110.
Zitt et al., Mometasone furoate nasal spray: a review of safety and systemic effects, Drug safety, 30:317-326, 2007.

## Claims

1. A pharmaceutical composition comprising a dual therapy comprising a therapeutically effective amount of the following active pharmaceutical ingredients :
i. a long acting β-agonist (LABA) which is salmeterol xinafoate, and
ii. a corticosteroid (CS) which is mometasone furoate;
wherein the pharmaceutical composition is formulated as a brittle matrix particle having a specific surface area, as measured using a surface area analyzer with 30% nitrogen in helium as adsorbate gas and with powder samples which are degassed for at least 2 hours at 30 °C using nitrogen prior to analysis, of 20 m²/g or greater.

2. The pharmaceutical composition of claim 1 further comprising one or more excipients.

3. The pharmaceutical composition of claim 2, wherein the excipient is a sugar, a sugar derivative selected from an aminosugar and a sugar alcohol, or an amino acid.

4. The pharmaceutical composition of claim 3, wherein the excipient is a sugar or a sugar derivative selected from lactose, mannitol, and trehalose, or the amino acid glycine.

5. The pharmaceutical composition according to any one of claims 1-4, wherein the dual therapy comprises a weight ratio of the long acting β-agonist to the corticosteroid from 1:0.1 to 1:100 in the composition, such as 5:22 of the long acting β-agonist to the corticosteroid.

6. The pharmaceutical composition according to any one of claims 1-5, wherein the pharmaceutical composition has a molar ratio of the dual therapy to the excipient of from 1:0 to 1:9 in the composition, such as 1:1 of the dual therapy to the excipient, or the pharmaceutical composition is formulated as a unit dose.

7. The pharmaceutical composition according to any one of claims 1-6, wherein the pharmaceutical composition is formulated for use in an inhaler, such as a metered dose inhaler, a dry powder inhaler, a single dose inhaler, multi-unit dose inhaler, nebulizer, or pressurized metered dose inhaler.

8. The pharmaceutical composition according to any one of claims 1-7, wherein the pharmaceutical composition has a specific surface area from 20 m²/g to 250 m²/g.

9. The pharmaceutical composition according to any one of claims 1-8, wherein the pharmaceutical composition is free of any impurities.

10. The pharmaceutical composition according to any one of claims 1-9 for use in treating or preventing a respiratory disease or disorder in a patient in need thereof, wherein the use comprises administering to the patient a therapeutically effective amount of the pharmaceutical composition.

11. The pharmaceutical composition for use of claim 10, wherein the respiratory disease or disorder is asthma or chronic obstructive pulmonary disease.

12. A method of preparing a brittle matrix pharmaceutical composition comprising:
(A) admixing two active pharmaceutical agents which are salmeterol xinafoate as a long acting β-agonist and momatesone furoate as a corticosteroid into a solvent wherein the solvent comprises an organic solvent and water to form a pharmaceutical composition wherein the pharmaceutical composition comprises an amount of the active pharmaceutical agents in the solvent from 0.01% to 10% (w/v);
(B) applying the pharmaceutical composition to a rotating surface wherein the surface is at a temperature from -70 °C to -120 °C; and
(C) freezing the pharmaceutical composition to form a brittle matrix pharmaceutical composition which has a specific surface area of 20 m²/g or greater.

13. The method claim 12, wherein the method further comprises lyophilizing the brittle matrix pharmaceutical composition.

14. The method according to claim 12 or 13, wherein the amount is from 0.01% (w/v) to 6% (w/v), preferably from 0.1% (w/v) to 5% (w/v).

## Patentansprüche

1. Ein Arzneimittel, umfassend eine duale Therapie, welche eine therapeutisch wirksame Menge der folgenden pharmazeutischen Wirkstoffe umfasst:
i. ein langwirkender β-Agonist (LABA), welcher Salmeterolxinofoat ist und
ii. ein Kortikosteroid (CS), welches Mometasonfuroat ist;
wobei das Arzneimittel formuliert ist als ein Teilchen mit spröder Matrix mit einer spezifischen Oberfläche, gemessen unter Verwendung eines Oberflächenanalysators mit 30% Stickstoff in Helium als Adsorbatgas und mit Pulverproben, welche für mindestens 2 Stunden bei 30 °C unter Verwendung von Stickstoff vor der Analyse entgast wurden, von 20 m²/g oder größer.

2. Das Arzneimittel gemäß Anspruch 1, ferner umfassend einen oder mehrere Exzipienten.

3. Das Arzneimittel gemäß Anspruch 2, wobei der Exzipient ein Zucker, ein Zuckerderivat, ausgewählt aus einem Aminozucker und einem Zuckeralkohol, oder eine Aminosäure ist.

4. Das Arzneimittel gemäß Anspruch 3, wobei der Exzipient ein Zucker oder ein Zuckerderivat, ausgewählt aus Laktose, Mannitol und Trehalose oder die Aminosäure Glycin ist.

5. Das Arzneimittel gemäß einem der Ansprüche 1-4, wobei die duale Therapie ein Gewichtsverhältnis des langwirkenden β-Agonisten zu dem Kortikosteroid von 1:0,1 bis 1:100 in der Zusammensetzung umfasst, wie 5:22 des langwirkenden β-Agonisten zum Kortikosteroid.

6. Das Arzneimittel gemäß einem der Ansprüche 1-5, wobei das Arzneimittel ein Molverhältnis der dualen Therapie zu dem Exzipienten von 1:0 bis 1:9 in der Zusammensetzung aufweist, wie 1:1 der dualen Therapie zu dem Exzipienten, oder das Arzneimittel als eine Einheitsdosis formuliert ist.

7. Das Arzneimittel gemäß einem der Ansprüche 1-6, wobei das Arzneimittel zur Verwendung in einem Inhalator, wie einem Dosieraerosol, einem Trockenpulverinhalator, einem Einzeldosis-Inhalator, Mehrdosen-Inhalator, Vernebler oder treibgasbetriebenem Dosieraerosol, formuliert ist.

8. Das Arzneimittel gemäß einem der Ansprüche 1-7, wobei das Arzneimittel eine spezifische Oberfläche von 20 m²/g bis 250 m²/g aufweist.

9. Das Arzneimittel gemäß einem der Ansprüche 1-8, wobei das Arzneimittel frei von jeglichen Verunreinigungen ist.

10. Das Arzneimittel gemäß einem der Ansprüche 1-9 zur Verwendung bei der Behandlung oder Vorbeugung einer Atemwegserkrankung oder -störung bei einem Patienten, der dieser bedarf, wobei die Verwendung das Verabreichen einer therapeutisch wirksamen Menge des Arzneimittels an den Patienten umfasst.

11. Das Arzneimittel zur Verwendung gemäß Anspruch 10, wobei die Atemwegserkrankung oder -störung Asthma oder chronisch obstruktive Lungenerkrankung ist.

12. Ein Verfahren zur Herstellung eines Arzneimittels mit spröder Matrix, umfassend:
(A) Mischen von zwei pharmazeutischen Wirkstoffen, bei denen es sich um Salmoterolxinafoat als einen langwirkenden β-Agonisten und Momatesonfuroat als ein Kortikosteroid handelt, in ein Lösungsmittel, wobei das Lösungsmittel ein organisches Lösungsmittel und Wasser umfasst, um ein Arzneimittel zu bilden, wobei das Arzneimittel eine Menge der pharmazeutischen Wirkstoffe in dem Lösungsmittel von 0,01% bis 10% (Gew./Vol.) umfasst;
(B) Aufbringen des Arzneimittels auf eine rotierende Oberfläche, wobei die Oberfläche eine Temperatur von -70 °C bis -120 °C aufweist und
(C) Gefrieren des Arzneimittels, um ein Arzneimittel mit spröder Matrix zu bilden, welches eine spezifische Oberflächen von 20 m²/g oder mehr aufweist.

13. Das Verfahren gemäß Anspruch 12, wobei das Verfahren ferner Lyophilisieren des Arzneimittels mit spröder Matrix umfasst.

14. Das Verfahren gemäß Anspruch 12 oder 13, wobei die Menge 0,01% (Gew./Vol.) bis 6% (Gew./Vol.), vorzugsweise 0,1% (Gew./Vol.) bis 5% (Gew./Vol.) beträgt.

## Revendications

1. Composition pharmaceutique comprenant une bithérapie comprenant une quantité efficace du point de vue thérapeutique des ingrédients pharmaceutiques actifs suivants :
i. un β-agoniste à action prolongée (LABA) qui est le xinafoate de salmétérol, et
ii. un corticostéroïde (CS) qui est le furoate de mométasone ;
laquelle composition pharmaceutique est formulée comme une particule à matrice fragile ayant une surface spécifique, telle que mesurée par utilisation d'un analyseur de superficie avec 30 % d'azote dans l'hélium en tant que gaz d'adsorbat et avec des échantillons de poudre qui sont dégazés pendant au moins 2 heures à 30°C par utilisation d'azote avant l'analyse, de 20 m²/g ou plus.

2. Composition pharmaceutique selon la revendication 1, comprenant en outre un ou plusieurs excipients.

3. Composition pharmaceutique selon la revendication 2, dans laquelle l'excipient est un sucre, un dérivé de sucre choisi parmi un amino-sucre et un alcool de sucre, ou un acide aminé.

4. Composition pharmaceutique selon la revendication 3, dans laquelle l'excipient est un sucre ou un dérivé de sucre choisi parmi le lactose, le mannitol et le tréhalose, ou l'acide aminé glycine.

5. Composition pharmaceutique selon l'une quelconque des revendications 1 à 4, dans laquelle la bithérapie comprend un rapport en poids du β-agoniste à action prolongée au corticostéroïde de 1/0,1 à 1/100 dans la composition, tel qu'un rapport du β-agoniste à action prolongée au corticostéroïde de 5/22.

6. Composition pharmaceutique selon l'une quelconque des revendications 1 à 5, laquelle composition pharmaceutique a un rapport molaire de la bithérapie à l'excipient de 1/0 à 1/9 dans la composition, tel qu'un rapport molaire de la bithérapie à l'excipient de 1/1, ou bien laquelle composition est formulée sous la forme d'une dose unitaire.

7. Composition pharmaceutique selon l'une quelconque des revendications 1 à 6, laquelle composition pharmaceutique est formulée pour être utilisée dans un inhalateur, tel qu'un inhalateur doseur, un inhalateur de poudre sèche, un inhalateur monodose, un inhalateur multidose, un nébuliseur, ou un inhalateur doseur pressurisé.

8. Composition pharmaceutique selon l'une quelconque des revendications 1 à 7, laquelle composition pharmaceutique a une surface spécifique de 20 m²/g à 250 m²/g.

9. Composition pharmaceutique selon l'une quelconque des revendications 1 à 8, laquelle composition pharmaceutique est exempte de quelconques impuretés.

10. Composition pharmaceutique selon l'une quelconque des revendications 1 à 9, pour une utilisation dans le traitement ou la prévention d'une maladie ou d'un trouble respiratoire chez un patient en ayant besoin, dans laquelle l'utilisation comprend l'administration au patient d'une quantité efficace du point de vue thérapeutique de la composition pharmaceutique.

11. Composition pharmaceutique pour une utilisation selon la revendication 10, dans laquelle la maladie ou le trouble respiratoire est l'asthme ou la broncho-pneumopathie chronique obstructive.

12. Méthode de préparation d'une composition pharmaceutique à matrice fragile comprenant :
(A) le mélange de deux agents pharmaceutiques actifs qui sont le xinafoate de salmétérol en tant que β-agoniste à action prolongée et le furoate de momatésone en tant que corticostéroïde dans un solvant, lequel solvant comprend un solvant organique et de l'eau, pour former une composition pharmaceutique, laquelle composition pharmaceutique comprend une quantité des agents pharmaceutiques actifs dans le solvant de 0,01 % à 10 % (p/v) ;
(B) l'application de la composition pharmaceutique à une surface rotative, laquelle surface est à une température de -70°C à -120°C ; et
(C) la congélation de la composition pharmaceutique pour former une composition pharmaceutique à matrice fragile qui a une surface spécifique de 20 m²/g ou plus.

13. Méthode selon la revendication 12, laquelle méthode comprend en outre la lyophilisation de la composition pharmaceutique à matrice fragile.

14. Méthode selon la revendication 12 ou 13, dans laquelle la quantité est de 0,01 % (p/v) à 6 % (p/v), de préférence de 0,1 % (p/v) à 5 % (p/v).
